# EUROPEAN PATENT APPLICATION

(11) **EP 4 201 357 A1**
(43) Date of publication of application: **28.06.2023**
(21) Application number: 22216420.4
(22) Date of filing: 23.12.2022
(51) Int. Cl.: A61B 18/14, A61B 5/287, A61B 5/00

(54) **CATHETER END EFFECTOR WITH RESILIENT FRAME AND FLEXIBLE INTERIOR**

(30) Priority: 27.12.2021 US 202163293879 P; 30.11.2022 US 202218071759
(71) Applicant: BIOSENSE WEBSTER (ISRAEL) LTD., 2066717 Yokneam (IL)
(72) Inventor: EBRAHIMI, Babak, Irvine 92618 (US); KNUDSON, John C., Irvine 92618 (US); BASU, Shubhayu, Irvine 92618 (US); VAN NIEKERK, Pieter E., Irvine 92618 (US)
(74) Representative: Carpmaels & Ransford LLP

(57) **Abstract**

An apparatus includes a catheter shaft assembly and an end effector. The end effector is configured to transition between a first configuration and a second configuration. The end effector is configured to fit within an outer sheath in the first configuration. The end effector is configured to expand outwardly away from the longitudinal axis in the second configuration when exposed distally relative to the distal end of the outer sheath. The end effector includes a resilient frame assembly that is configured to resiliently bias the end effector toward the second configuration. The end effector further includes a first flex circuit assembly secured to the resilient frame assembly. The first flex circuit assembly includes a first flexible substrate and a first plurality of electrodes positioned on the first flexible substrate. The electrodes are configured to pick up electrical potentials from tissue or blood or ablate tissue.

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of electrical energy (e.g., radiofrequency (AC type) or irreversible electroporation (IRE), such as pulsed field (DC type) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with electrical energy to thereby ablate the contacted tissue. In some cases, the electrodes may be bipolar. In some other cases, a monopolar electrode may be used in conjunction with a ground pad or other reference electrode that is in contact with the patient. Irrigation may be used to draw heat from ablating components of an ablation catheter; and to prevent the formation of blood clots near the ablation site.

Examples of ablation catheters are described in U.S. Pat. No. 10,743,932, entitled "Integrated Ablation System using Catheter with Multiple Irrigation Lumens," issued August 18, 2020, the disclosure of which is incorporated by reference herein, in its entirety; U.S. Pat. No. 10,660,700, entitled "Irrigated Balloon Catheter with Flexible Circuit Electrode Assembly," issued May 26, 2020, the disclosure of which is incorporated by reference herein, in its entirety; U.S. Pub. No. 2018/0071017, entitled "Ablation Catheter with a Flexible Printed Circuit Board," published March 15, 2018, the disclosure of which is incorporated by reference herein, in its entirety; U.S. Pat. No. 10,702,177, entitled "Catheter with Bipole Electrode Spacer and Related Methods," issued July 7, 2020, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; and U.S. Pat. No. 9,801,585, entitled "Electrocardiogram Noise Reduction," issued October 31, 2017, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pat. No. 10,702,177, entitled "Catheter with Bipole Electrode Spacer and Related Methods," issued July 7, 2020, the disclosure of which is incorporated by reference herein, in its entirety.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein, in its entirety; and various other references that are cited herein.

While several catheter systems and methods have been made and used, it is believed that no one prior to the inventors has made or used the invention described in the appended claims.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2A depicts a perspective view of the catheter assembly of FIG. 1, with an end effector of the catheter in a proximal position relative to an outer sheath of the catheter;
FIG. 2B depicts a perspective view of the catheter assembly of FIG. 1, with the end effector in a distal position relative to the outer sheath;
FIG. 3 depicts a top plan view of another end effector that may be incorporated into the catheter assembly of FIG. 1;
FIG. 4 depicts a perspective view of the end effector of FIG. 3;
FIG. 5 depicts an exploded perspective view of the end effector of FIG. 3;
FIG. 6 depicts a top plan view of another end effector that may be incorporated into the catheter assembly of FIG. 1;
FIG. 7 depicts a top plan view of another end effector that may be incorporated into the catheter assembly of FIG. 1;
FIG. 8 depicts a top plan view of another end effector that may be incorporated into the catheter assembly of FIG. 1;
FIG. 9 depicts a top plan view of another end effector that may be incorporated into the catheter assembly of FIG. 1; and
FIG. 10 depicts a top plan view of another end effector that may be incorporated into the catheter assembly of FIG. 1.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "generally," "substantially," "about," or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

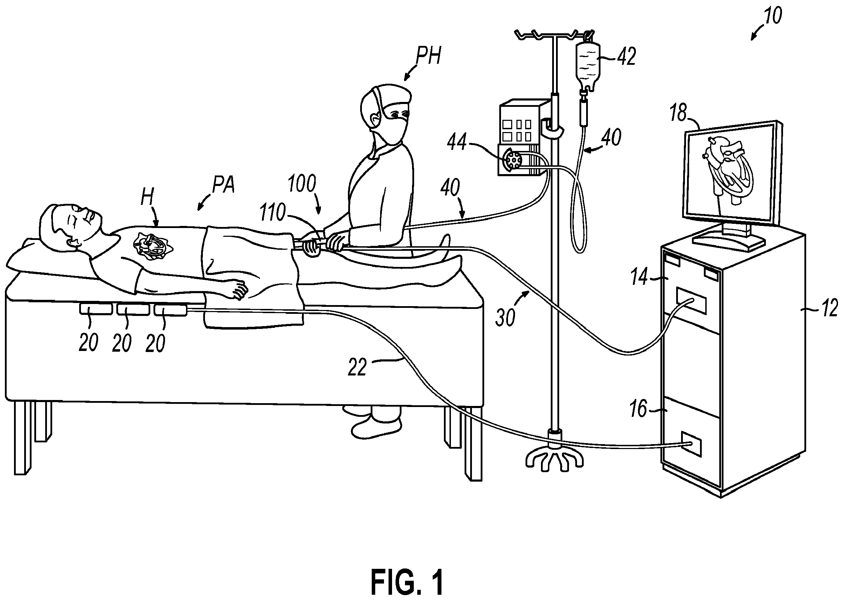

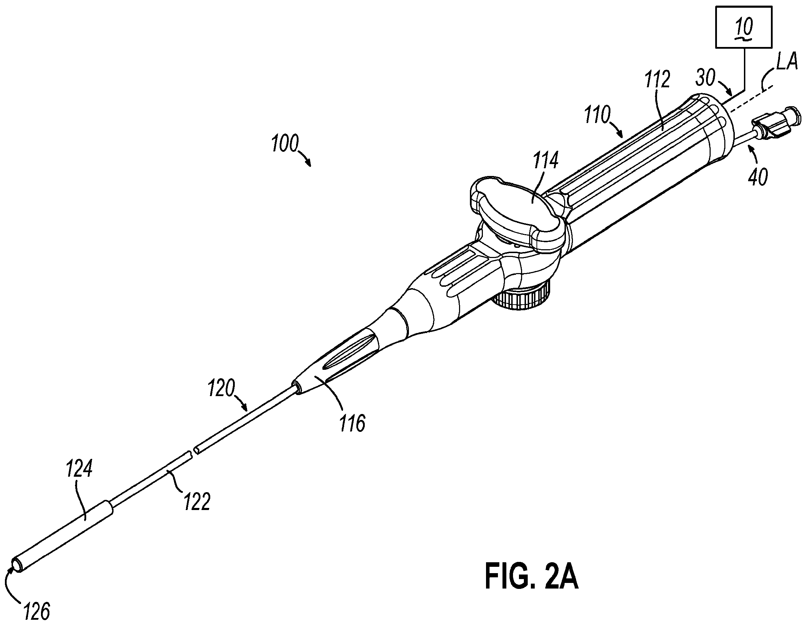

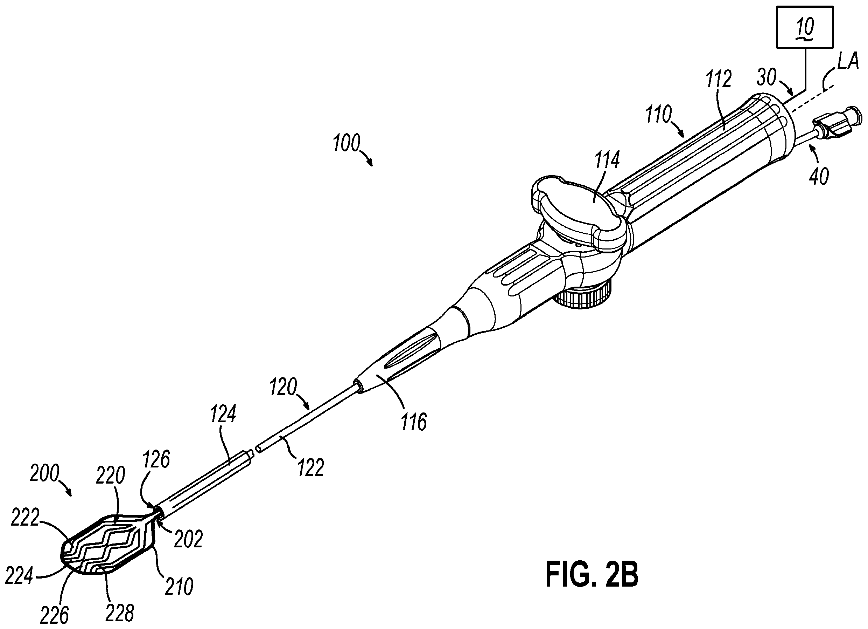

FIG. 1 shows an exemplary medical procedure and associated components of a cardiac catheter system that may be used to provide EP mapping or cardiac ablation as referred to above. In particular, FIG. 1 shows a physician (PH) grasping a handle assembly (110) of a catheter assembly (100), with an end effector (200) (shown in FIG. 2B but not shown in FIG. 1) of a flexible catheter (120) (shown in FIGS. 2A-2B but not shown in FIG. 1) of catheter assembly (100) disposed in a patient (PA) to map potentials in tissue or ablate tissue in or near the heart (H) of the patient (PA). As shown in FIGS. 2A-2B, catheter assembly (100) includes handle assembly (110), catheter (120) extending distally from handle assembly (110), end effector (200) located at a distal end of catheter (120), and a deflection drive actuator (114) associated with handle assembly (110). Deflection drive actuator (114) is rotatable relative to a casing (112) of handle assembly (110) to thereby deflect end effector (140) and a distal portion of catheter (120) away from a central longitudinal axis (LA) defined by a proximal portion of catheter (120). Various suitable components that may be coupled with deflection drive actuator (114) and catheter (120) to provide such functionality will be apparent to those skilled in the art in view of the teachings herein.

Catheter (120) includes an elongate flexible shaft (122) and an outer sheath (124). Shaft (122) is coaxially and slidably disposed within outer sheath (124). End effector (200) is positioned at the distal end of shaft (122). The proximal end of catheter (120) extends distally from a nozzle member (116) of handle assembly (110). In some versions, outer sheath (124) is an integral component of catheter (120). In some other versions, sheath (124) is a component of another instrument; and catheter (120) is inserted into sheath (124). In either scenario, shaft (122) and an outer sheath (124) may transition between two arrangements, including a first arrangement as shown in FIG. 2A where outer sheath (124) is distally positioned in relation to shaft (122), such that end effector (200) is contained within outer sheath (124); and a second arrangement as shown in FIG. 2B where outer sheath (124) is proximally positioned in relation to shaft (122), such that end effector (200) is exposed relative to outer sheath (124). In some versions, shaft (122) translates relative to handle assembly (110) to achieve the different longitudinal positioning relative to outer sheath (124). In some other versions, outer sheath (124) translates relative to handle assembly (110) to achieve the different longitudinal positioning relative to shaft (122).

Catheter (120) and end effector (200) may be in the state shown in FIG. 2A when catheter (120) is introduced into the body of the patient (PA); and during transit from the insertion site to the targeted cardiovascular region within the patient (PA). Once catheter (120) and end effector (200) reach the targeted cardiovascular region within the patient (PA), outer sheath (124) may be retracted proximally to expose end effector (200), thereby allowing end effector (200) to achieve the expanded, deployed state shown in FIG. 2B.

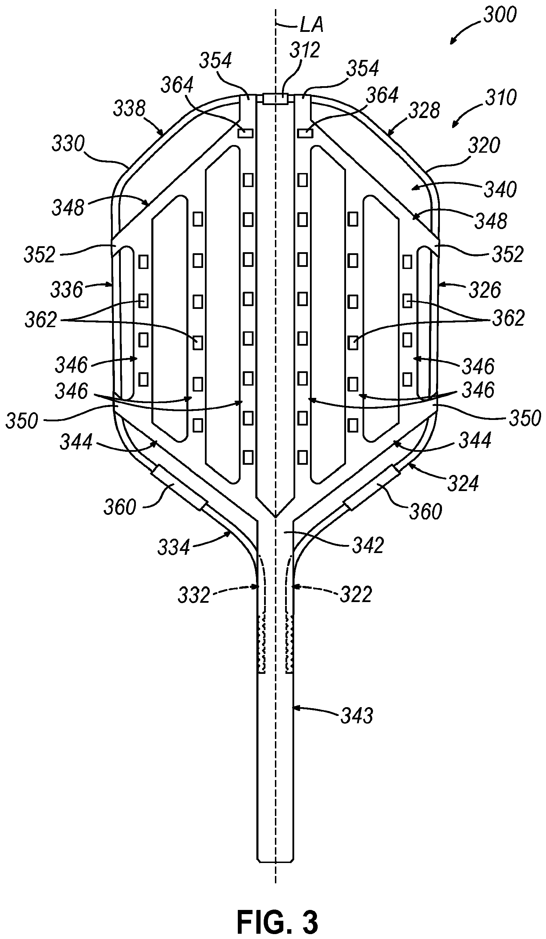

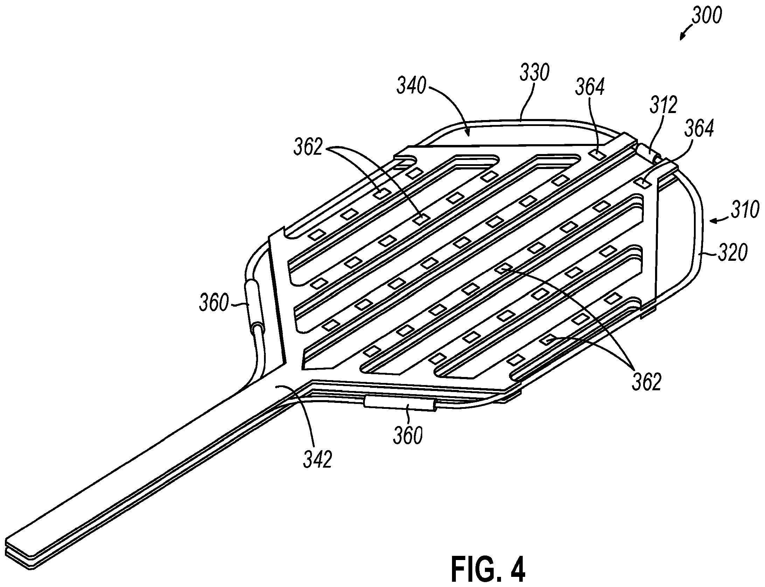

As shown in FIG. 2B, end effector (200) of the present example includes a resilient wire frame (210) and a flex circuit assembly (220) secured to wire frame (210). A proximal end (202) of end effector (200) is fixedly secured to the distal end of shaft 122). Flex circuit assembly (220) includes a plurality of elongate portions (220, 222, 224, 226, 228) that span across the width and length of wire frame (210). Elongate portions (220, 222, 224, 226, 228) may include EP mapping electrodes, ablation electrodes, position sensors, temperature sensors, and/or various other components. Examples of different features and configurations that may be incorporated into end effector (200) will be described in greater detail below with reference to FIGS. 3-10.

Catheter assembly (100) is coupled with a guidance and drive system (10) via a cable (30). Catheter assembly (100) is also coupled with a fluid source (42) via a fluid conduit (40), though this is optional. A set of field generators (20) are positioned underneath the patient (PA) and are also coupled with guidance and drive system (10) via a cable (22). Guidance and drive system (10) of the present example includes a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via pairs of electrodes of end effector (200) as described in greater detail below. Console (12) includes a processor (not shown) that processes such EP mapping signals and thereby provides EP mapping as is known in the art. In addition, or in the alternative, first driver module (14) may be operable to provide electrical energy (e.g., RF or IRE) to ablation electrodes of end effector (200) to thereby ablate tissue.

In some versions, first driver module (14) is also operable to receive position indicative signals from one or more position sensors in end effector (200), as will be described in greater detail below. In such versions, the processor of console (12) is also operable to process the position indicative signals from position sensors to thereby determine the position of end effector (200) of catheter (120) within the patient (PA). Other components and techniques that may be used to generate real-time position data associated with end effector (200) may include wireless triangulation, acoustic tracking, optical tracking, inertial tracking, and the like. By way of further example only, alternative position sensing may include impedance measurement-based position sensing. Examples of such position sensing are described in U.S. Pat. No. 7,869,865, entitled "Current-Based Position Sensing," issued January 11, 2011, the disclosure of which is incorporated by reference herein, in its entirety; and U.S. Pat. No. 8,456,182, entitled "Current Localization Tracker," issued June 4, 2013, the disclosure of which is incorporated by reference herein, in its entirety. Alternatively, any other suitable position sensing technologies and techniques may be used.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from position sensors of end effector (200). For instance, as end effector (200) of catheter (120) moves within the patient (PA), the corresponding position data from position sensors may cause the processor of console (12) to update the patient anatomy views in display (18) in real time to depict the regions of patient anatomy around end effector (200) as end effector (200) moves within the patient (PA). Moreover, the processor of console (12) may drive display (18) to show locations of aberrant conductive tissue sites, as detected via EP mapping with end effector (200). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (200) on the images of the patient's anatomy, such as by superimposing an illuminated dot, a crosshair, a graphical representation of end effector (200), or some other form of visual indication. Such a superimposed visual indication may also move within the images of the patient anatomy on display (18) in real time as the physician moves end effector (200) within the patient (PA), thereby providing real-time visual feedback to the operator about the position of end effector (200) within the patient (PA) as end effector (200) moves within the patient (PA). The images provided through display (18) may thus effectively provide a video tracking the position of end effector (200) within a patient (PA), without necessarily having any optical instrumentation (i.e., cameras) viewing end effector (200). In the same view, display (18) may simultaneously visually indicate the locations of aberrant conductive tissue sites detected through the EP mapping. The physician (PH) may thus view display (18) to observe the real time positioning of end effector (200) in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). In some variations, conduit (40), fluid source (42), and pump (44) are omitted entirely. In versions where these components are included, end effector (200) may be configured to communicate irrigation fluid from fluid source (42) to the target site in the patient. Such irrigation may be provided in accordance with the teachings of any of the various patent references cited herein; or in any other suitable fashion as will be apparent to those skilled in the art in view of the teachings herein.

### II. Examples of End Effector with Resilient Frame and Flexible Interior

In an EP mapping end effector, it may be desirable to configure the end effector to promote contact between EP mapping electrodes of the end effector and tissue of anatomical structures within the cardiovascular system (e.g., within chambers of the heart (H), within the pulmonary vein, etc.). Similarly, it may be desirable to configure the end effector to maximize the number of EP mapping electrodes of the end effector in contact with tissue of anatomical structures within the cardiovascular system. To the extent that the geometries of these anatomical structures vary within each patient, it may be desirable for the end effector to have sufficient flexibility to conform to the different geometries of these anatomical structures. It may also be desirable to impart a certain degree of resilience to the end effector to ensure that the end effector sufficiently expands upon deployment from outer sheath (124). It may therefore be desirable for the end effector to strike an appropriate balance between resilience and conformal flexibility. Examples of end effectors that may strike this balance will be described in greater detail below. It should be understood that the end effectors described below may be readily incorporated into catheter assembly (100), such that each of the end effectors described below may be understood as variations of end effector (200).

### A. Example of End Effector with Resilient Frame and Resilient Flexible Interior

FIGS. 3-5 show an end effector (300) that includes a resilient frame assembly (310) and a pair of flex circuit assemblies (340). End effector (300) is deformable to fit entirely within outer sheath (124) when outer sheath (124) is distally positioned in relation to shaft (122). End effector (300) is also configured to achieve an expanded configuration when outer sheath (124) is proximally positioned in relation to shaft (122). In the expanded configuration, end effector (300) is substantially flat such that end effector (300) extends along a substantially flat plane along which the longitudinal axis (LA) of catheter (120) extends. End effector (300) is symmetric about the longitudinal axis (LA) of catheter (120) in this example. In some other versions, end effector (300) is asymmetric about the longitudinal axis (LA) of catheter (120).

Resilient frame assembly (310) includes a first wire (320) and a second wire (330). Wires (320, 320) are configured to resiliently bias end effector (300) to the flat, expanded configuration shown in FIGS. 3-4; while still allowing end effector (300) to compress to fit within outer sheath (124). In the present example, each wire (320, 330) is formed of nitinol, though any other suitable kind(s) of material(s) may be used to form each wire (320, 330). First wire (320) includes a tail portion (322), a proximal portion (324), a longitudinally extending portion (326), and a distal portion (328). Second wire (330) includes a tail portion (332), a proximal portion (334), a longitudinally extending portion (336), and a distal portion (338). Distal portions (328, 338) of wires (320, 330) are joined together by a union sleeve (312). In some other versions, resilient frame assembly (310) is formed by a single piece of wire such that union sleeve (312) is omitted. Tail portions (322, 332) of wires (320, 330) are configured to be secured to the distal end of shaft (122), such that end effector (300) is structurally secured to shaft (122) via tail portions (322, 332). Alternatively, any other suitable structures may be used to secure end effector (300) to shaft (122).

In the present example, proximal portions (324, 334) are oriented obliquely relative to the longitudinal axis (LA). This oblique orientation may promote collapse of end effector (300) when outer sheath (124) is advanced distally over end effector (300). In other words, the oblique orientation of proximal portions (324, 334) may promote a smooth transition for end effector (300) from the deployed, expanded state (e.g., similar to what is shown in FIG. 2B) to the contained, contracted state (e.g., similar to what is shown in FIG. 2A).

Flex circuit assembly (340) includes a substrate (342) that defines a proximal pair of obliquely extending portions (344), a distal pair of obliquely extending portions (348), and a plurality of longitudinally extending portions (346). Proximal obliquely extending portions (344) diverge distally away from the longitudinal axis (LA) and form oblique angles with the longitudinal axis (LA). Distal obliquely extending portions (348) converge distally toward the longitudinal axis (LA) and form oblique angles with the longitudinal axis (LA). Longitudinally extending portions (346) extend between obliquely extending portions (344, 348) and are parallel with the longitudinal axis (LA).

Substrate (342) is secured to resilient frame assembly (310) via attachment points (350, 352). Attachment points (350) are located near the intersection of the outermost longitudinally extending portions (346) and proximal obliquely extending portions (344). Attachment points (352) are located near the intersection of the outermost longitudinally extending portions (346) and distal obliquely extending portions (348). By way of example only, substrate (342) may be secured to resilient frame assembly (310) at attachment points (350, 352) via adhesive or epoxy, via welding, or using any other suitable kinds of components or techniques.

Flex circuit assembly (340) of the present example further includes a plurality of electrodes (362). Electrodes (362) are arranged in pairs along longitudinally extending portions (346) in this example. In some versions, electrodes (362) are also positioned along obliquely extending portions (344, 348). Each pair of electrodes (362) is operable to provide bipolar EP mapping by picking up electrocardiogram signals from tissue as is known in the art. By way of example only, the spacing of electrodes (362) in each pair of electrodes (362) may be arranged to conform to the arrangements shown and described in U.S. Pub. No. 2020/0297281, entitled "Electrode Configurations for Diagnosis of Arrhythmias," published September 24, 2020, the disclosure of which is incorporated by reference herein, in its entirety. Signals picked up by electrodes (362) may be communicated back through electrical conduits (not shown) in catheter (120) to console (12), which may process the signals to provide EP mapping to thereby identify locations of aberrant electrical activity within the cardiac anatomy. This may in turn allow the physician (PH) to identify the most appropriate regions of cardiac tissue to ablate (e.g., with RF energy, IRE energy, cryoablation, etc.), to thereby prevent or at least reduce the communication of aberrant electrical activity across the cardiac tissue.

Electrodes (362) may be formed of platinum, gold, or any other suitable material. Electrodes (362) may include various coatings, if desired. For instance, electrodes (362) may include a coating that is selected to improve the signal-to-noise ratio of signals from pairs of electrodes (362). Such coatings may include, but need not be limited to, iridium oxide (IrOx) coating, poly(3,4-ethylenedioxythiophene) (PEDOT) coating, Electrodeposited Iridium Oxide (EIROF) coating, Platinum Iridium (PtIr) coating, or any other suitable coating.

In some versions, electrodes (362) may be provided on substrate (342) as a thin film through a physical vapor deposition (PVD) process. By way of example only, such a PVD process may be carried out in accordance with at least some of the teachings of International Patent Pub. No. WO 2015/117908, entitled "Medical Device for Ablating Tissue Cells and System Comprising a Device of This Type," published August 13, 2015, the disclosure of which is incorporated by reference herein, in its entirety; at least some of the teachings of German Patent Pub. No. 102017130152, entitled "Method for Operating a Multi-Layer Structure," published January 3, 2019, the disclosure of which is incorporated by reference herein, in its entirety; or at least some of the teachings of U.S. Pat. No. 10,061,198, entitled "Method for Producing a Medical Device or a Device with Structure Elements, Method for Modifying the Surface of a Medical Device or of a Device with Structure Elements, Medical Device and Laminated Composite with a Substrate," published August 28, 2018, the disclosure of which is incorporated by reference herein, in its entirety. Other methods may also be employed to provide electrodes (362), conductive traces, or other circuit components on substrate (342), including but not limited to sputter deposition, chemical vapor deposition (CVD), thermal deposition, etc.

Substrate (342) may be formed of polyimide, polyether ether ketone, or any other suitable flex circuit substrate material(s); with electrodes (362) and associated conductive traces being printed on substrate (342). In some versions, substrate (342) is formed of a resilient material such as nitinol to assist resilient frame assembly (310) in resiliently biasing end effector (300) to the expanded configuration shown in FIGS. 3-4 when end effector (300) is exposed relative to outer sheath (124). As another variation, substrate (342) may include a polymeric layer laid over a resilient (e.g., nitinol) layer.

Flex circuit assembly (340) of the present example further includes a pair of temperature sensors (364). By way of example only, each temperature sensor (364) may comprise a thermocouple and/or any other suitable kind of configuration. Temperature sensors (364) are positioned at the intersections of the innermost longitudinally extending portions (346) and distal portions (348) in the present example. However, other versions may provide one or more temperature sensors (364) at any other suitable location(s) on end effector (300), in addition to or in lieu of the locations shown in FIG. 3. Each temperature sensor (364) is configured to sense the temperature at the corresponding region of end effector (300). Temperature data from temperature sensors (364) may be communicated to console (12), which may factor such temperature into one or more control algorithms in any suitable fashion. By way of example only, temperature data from temperature sensors (364) may be utilized to further refine EP signal data from electrodes (362). In addition, or in the alternative, in versions where end effector (300) includes one or more ablation electrodes, temperature data from temperature sensors (364) may be utilized to regulate the delivery of power to such ablation electrodes, to thereby prevent overheating of end effector (300) and/or overheating of adjacent tissue. While two temperature sensors (364) are shown in FIG. 3, other variations of end effector (300) may include more than two temperature sensors (364); just one temperature sensor (364); or no temperature sensor (364) at all.

End effector (300) of the present example further includes a pair of position sensors (360). One position sensor (360) is positioned on proximal portion (324) of wire (320) while the other position sensor (360) is positioned on proximal portion (334) of wire (330). In addition, or in the alternative, one or more position sensors (360) may be positioned along longitudinally extending portions (326, 336), along distal portions (328, 338), on substrate (322), and/or elsewhere on end effector (300). Each position sensor (360) is operable to generate signals that are indicative of the position and orientation of the corresponding portion of end effector (300) within the patient (PA). Position sensors (360) generate electrical signals in response to the presence of an alternating electromagnetic field generated by field generators (20). Each position sensor (360) may be coupled with corresponding wire, a corresponding trace, or any other suitable corresponding electrical conduit along or otherwise through catheter (120), thereby enabling signals generated by position sensor (360) to be communicated back through electrical conduits (not shown) in catheter (120) to console (12). Console (12) may process the signals from position sensor (360) to identify the positions of the corresponding portions of end effector (300) within the patient (PA).

Other components and techniques that may be used to generate real-time position data associated with end effector (300) may include wireless triangulation, acoustic tracking, optical tracking, inertial tracking, and the like. In some versions, position sensors (360) may be omitted. In versions providing position sensors (360) or other position tracking features, the position data may be correlated with the EP mapping data picked up by electrodes (362) such that console (12) may store the locations of EP signals in conjunction with the precise anatomical locations where such EP signals were generated within the patient (PA).

Substrate (342) of the present example further includes a tail portion (343). Tail portion (343) of substrate (342) is adjacent to tail portions (322, 332) of wires (320, 330) and is configured to fit in or on the distal end of shaft (122). In some versions, tail portion (343) includes a plurality of termination pads (not shown) that are in electrical communication with electrodes (362) and temperature sensors (364) via corresponding traces (not shown) formed in flex circuit assembly (340). Wires (not shown) may be coupled with such termination pads and extend along the interior of catheter (120) to provide a path for electrical communication from electrodes (362) and temperature sensors (364) to console (12). In some other versions, a flex circuit extends along the length of catheter (120) to provide a path for electrical communication from electrodes (362) and temperature sensors (364) to console (12). For instance, tail portion (343) may extend along the length of catheter (120) and include traces that provide a path for electrical communication from electrodes (362) and temperature sensors (364) to console (12).

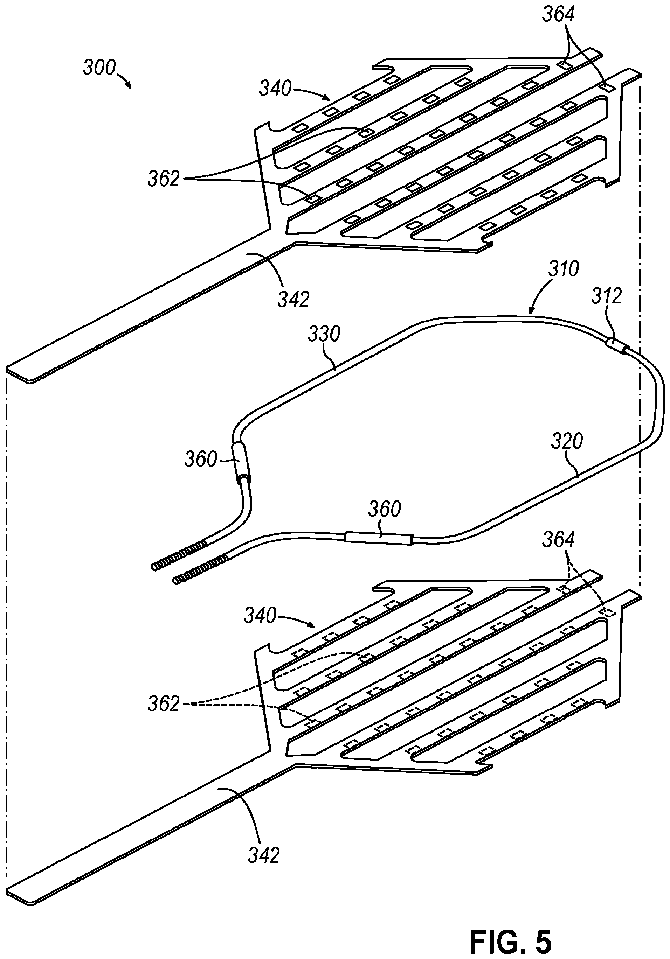

As best seen in FIG. 5, end effector (300) of the present example includes one flex circuit assembly (340) on one side of resilient frame assembly (310) and another flex circuit assembly (340) on the other side of resilient frame assembly (310). These flex circuit assemblies (340) may be configured and operable identically to each other; yet flex circuit assemblies (340) are oriented such that their respective electrodes (362) and temperature sensors (364) face in opposite directions. In other words, electrodes (362) and temperature sensors (364) of one flex circuit assembly (340) may face upwardly in the view shown in FIG. 5 while electrodes (362) and temperature sensors (364) of the other flex circuit assembly (340) may face downwardly in the view shown in FIG. 5. End effector (300) may thus present electrodes (362) and temperature sensors (364) on opposing faces of end effector (300) when end effector (300) is in a fully assembled state. In some other versions, end effector (300) may provide electrodes (363) and temperature sensors (364) on opposite surfaces of the same single substrate (342), such that two separate flex circuit assemblies (340) are not joined to resilient frame assembly (310).

In an example of a method where end effector (300) is used in the patient (PA), the physician (PH) may initially insert catheter (120) into a major vein or artery (e.g., the femoral artery) and then advance catheter (120) to position end effector (300) in the heart (H) of the patient (PA). During this advancement of catheter (120), console (12) may track the position of catheter (120) in real time based on signals from position sensors (360) and render the real-time position on display (18), such that the physician (PH) may observe display (18) to determine the real-time position of catheter (120) (e.g., in relation to a preoperatively obtained image of the patient (PA)). Outer sheath (124) may be in the distal position (FIG. 2A) during the advancement of catheter (120) into the patient (PA).

Once the distal end of catheter (120) is suitably positioned, the physician (PH) may retract outer sheath (124) proximally to uncover end effector (300). End effector (300) may then reach the expanded state as shown in FIGS. 3-4. With end effector (200) in the expanded state, the physician (PH) may press end effector (300) against anatomical structures in the cardiac system of the patient (PA), such as the wall of the pulmonary vein, the wall of an atrium or other chamber of the heart (H), etc. In some procedures, the physician (PH) may apply end effector (300) against the tissue in a brushing motion, in a stamping motion, and/or using any other suitable technique(s). As end effector (300) engages these anatomical structures, electrodes (362) that are in contact with the tissue will pick up signals from the tissue, thereby enabling console (12) to perform EP mapping. Console (12) may correlate the signals from electrodes (362) with contemporaneous signals from position sensors (360) to thereby enable storage of the precise locations of aberrant electrical signals within the cardiac tissue of the patient (PA). Console (12) may thus enable mapping of aberrant electrical signal sites with respect to a preoperatively obtained image of the cardiac anatomy of the patient (PA). With such a map being established, the physician (PH) may then provide precisely targeted ablation (e.g., using end effector (300) or some other device) or some other kind of targeted therapy based on the map.

The flexibility of end effector (300) may allow end effector (300) to conform to the contours and other surface geometry of cardiac tissue when end effector (300) is pressed against cardiac tissue (e.g., within a chamber of the heart (H), within the pulmonary vein, etc.). The deformation of end effector (300) may promote full contact between electrodes (362) and cardiac tissue. In some scenarios, only one side of end effector (300) will be contacting tissue at any given moment of operation where end effector (300) is contacting tissue. In other words, either one or more pairs of electrodes (362) of only one flex circuit assembly (340) may be contacting tissue, without any electrodes (362) of the other flex circuit assembly (340) contacting tissue. The electrodes (362) of end effector (300) that are not contacting tissue may nevertheless be contacting the blood that is circulating through the cardiovascular system of the patient (PA). Any electrodes (362) of end effector (300) that are contacting blood may serve as reference electrodes, picking up reference potentials from the blood to reduce noise or far field signals, as is known in the art.

While only EP mapping electrodes (362) are shown on end effector (300) in the present example, other versions of end effector (300) may include one or more ablation electrodes in addition to, or in lieu of, including EP mapping electrodes (362). Such ablation electrodes may be used to apply electrical energy to tissue that is in contact with the ablation electrodes, to thereby ablate the tissue. Each ablation electrode may be coupled with a corresponding trace or other electrical conduit of end effector (300), thereby enabling console (12) to communicate electrical energy through electrical conduits (not shown) in catheter (120) to the traces or other conduits of end effector (300) to reach the ablation electrodes. Such electrical energy may include radiofrequency (AC type) electrical energy, pulsed field (DC type) electrical energy (e.g., irreversible electroporation, etc.), or some other form of electrical energy. Alternatively, ablation electrodes may be omitted from end effector (300). As yet another variation, some versions of electrodes (362) may be configured to alternative between functionalities, including an EP mapping functionality and an ablation functionality. In some versions where electrodes (362) provide ablation functionality in addition to providing EP mapping functionality, more than two electrodes (362) may be activated simultaneously to provide ablation, such that electrodes (362) may be activated in groups to collectively provide an equivalent to an ablation electrode that is larger in size than one single electrode (362) or pair of electrodes (362).

### B. End Effector with Resilient Frame and Non-Extensible Flexible Interior

As noted above in the description of end effector (300), a flex circuit of an end effector may provide additional resilience to an end effector to supplement the resilience provided by a wire frame of the end effector. In some scenarios, it may be desirable for a flex circuit of an end effector to simply provide flexibility without necessarily providing additional resilience to the end effector to supplement the resilience provided by a wire frame of the end effector. In some such scenarios, it may also be desirable for the flex circuit to be non-extensible. In versions where the flex circuit is non-extensible, it may be desirable to configure the flex circuit to provide a desired degree of flexibility to promote conformance with the different geometries of different anatomical structures within a cardiovascular system. Examples of end effectors providing such functionality are described in greater detail below.

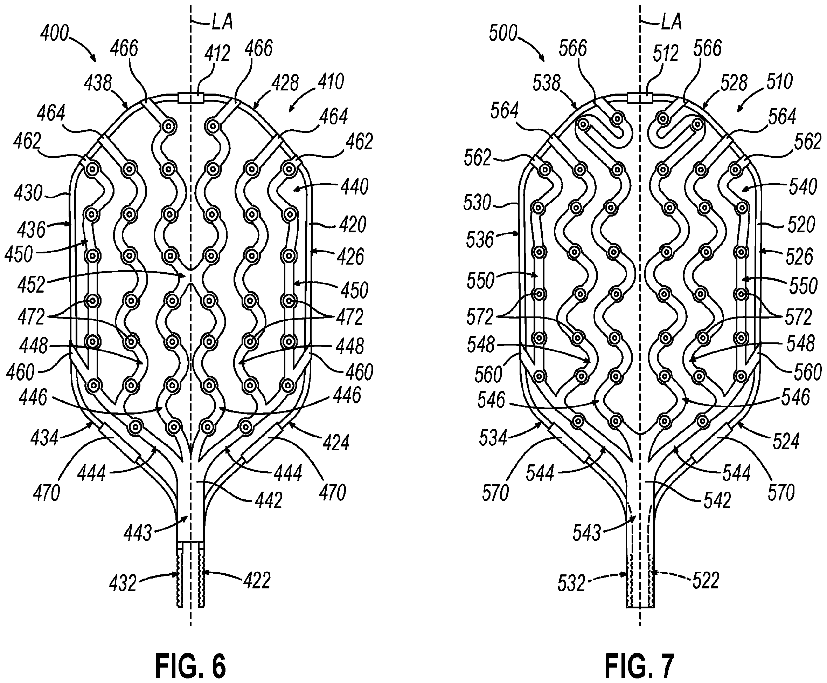

FIG. 6 shows an end effector (400) that includes a resilient frame assembly (410) and a flex circuit assembly (440). Except as otherwise described below, end effector (400) may be configured and operable just like end effector (300) described above. End effector (400) is thus deformable to fit entirely within outer sheath (124) when outer sheath (124) is distally positioned in relation to shaft (122). End effector (400) is also configured to achieve an expanded configuration when outer sheath (124) is proximally positioned in relation to shaft (122). In the expanded configuration, end effector (400) is substantially flat such that end effector (400) extends along a substantially flat plane along which the longitudinal axis (LA) of catheter (120) extends. End effector (400) is symmetric about the longitudinal axis (LA) of catheter (120) in this example. In some other versions, end effector (400) is asymmetric about the longitudinal axis (LA) of catheter (120).

Resilient frame assembly (410) includes a first wire (420) and a second wire (430). Wires (420, 420) are configured to resiliently bias end effector (400) to the flat, expanded configuration shown in FIG. 6; while still allowing end effector (400) to compress to fit within outer sheath (124). In the present example, each wire (420, 430) is formed of nitinol, though any other suitable kind(s) of material(s) may be used to form each wire (420, 430). First wire (420) includes a tail portion (422), a proximal portion (424), a longitudinally extending portion (426), and a distal portion (428). Second wire (430) includes a tail portion (432), a proximal portion (434), a longitudinally extending portion (436), and a distal portion (438). Distal portions (428, 438) of wires (420, 430) are joined together by a union sleeve (412). In some other versions, resilient frame assembly (410) is formed by a single piece of wire such that union sleeve (412) is omitted. Tail portions (422, 432) of wires (420, 430) are configured to be secured to the distal end of shaft (122), such that end effector (400) is structurally secured to shaft (122) via tail portions (422, 432). Alternatively, any other suitable structures may be used to secure end effector (400) to shaft (122).

In the present example, proximal portions (424, 434) are oriented obliquely relative to the longitudinal axis (LA). This oblique orientation may promote collapse of end effector (400) when outer sheath (124) is advanced distally over end effector (400). In other words, the oblique orientation of proximal portions (424, 434) may promote a smooth transition for end effector (400) from the deployed, expanded state (e.g., similar to what is shown in FIG. 2B) to the contained, contracted state (e.g., similar to what is shown in FIG. 2A).

Flex circuit assembly (440) includes a substrate (442) that defines a proximal pair of obliquely extending portions (444), an inner pair of longitudinally extending portions (446), an intermediate pair of longitudinally extending portions (448), and an outer pair of longitudinally extending portions (450). Obliquely extending portions (444) diverge distally away from the longitudinal axis (LA) and form oblique angles with the longitudinal axis (LA). Longitudinally extending portions (446, 448, 450) extend between obliquely extending portions (444) of substrate (442) and distal portions (428, 438) of wires (420, 430). Each longitudinally extending portion (446, 448, 450) has one or more regions that curve laterally toward or away from the longitudinal axis (LA), such that at least a portion of each longitudinally extending portion (446, 448, 450) has a serpentine configuration. In the present example, inner longitudinally extending portions (446) are joined together at a junction (452).

Substrate (442) is secured to resilient frame assembly (410) via attachment points (460, 462, 464, 466). Attachment points (460) are located at distal ends of obliquely extending portions (444). Attachment points (462) are located at distal ends of outer longitudinally extending portions (450). Attachment points (464) are located at distal ends of intermediate longitudinally extending portions (448). Attachment points (466) are located at distal ends of inner longitudinally extending portions (446). By way of example only, substrate (442) may be secured to resilient frame assembly (410) at attachment points (460, 462, 464, 466) via adhesive or epoxy, via welding, or using any other suitable kinds of components or techniques.

Flex circuit assembly (440) of the present example further includes a plurality of electrodes (472). Electrodes (472) are arranged in pairs along longitudinally extending portions (446, 448, 450) in this example. In some versions, electrodes (472) are also positioned along obliquely extending portions (444). Electrodes (472) may be configured and operable just like electrodes (362) described above, such that each pair of electrodes (472) may be operable to provide bipolar EP mapping by picking up electrocardiogram signals from tissue as is known in the art. Similarly, electrodes (472) may be provided on substrate (442) using any of the techniques described above in the context of electrodes (362) and substrate (342).

In the present example, substrate (442) is formed of a material that is flexible yet non-extensible. Substrate (442) may be formed of polyimide, polyether ether ketone, or any other suitable flex circuit substrate material(s); with electrodes (472) and associated conductive traces being printed on substrate (442). In some versions, substrate (442) is formed of a resilient material such as nitinol to assist resilient frame assembly (410) in resiliently biasing end effector (400) to the expanded configuration shown in FIG. 6 when end effector (400) is exposed relative to outer sheath (124). As another variation, substrate (442) may include a polymeric layer laid over a resilient (e.g., nitinol) layer.

It should be understood that, with at least a portion of each longitudinally extending portion (446, 448, 450) having a serpentine configuration as described above, this configuration may allow longitudinally extending portions (446, 448, 450) to deform to a greater degree than longitudinally extending portions (446, 448, 450) might otherwise deform in versions where longitudinally extending portions (446, 448, 450) would be straight. In other words, the serpentine structural aspects of longitudinally extending portions (446, 448, 450) enhance the deformability of longitudinally extending portions (446, 448, 450). This enhanced deformability may further enhance the ability of electrodes (472) to reach tissue in different geometries of anatomical structures. Thus, the combination of flexible material forming substrate (442) and the serpentine structural aspects of longitudinally extending portions (446, 448, 450) may promote maximum contact between electrodes (472) and different geometries of anatomical structures.

While not shown in FIG. 6, some versions of flex circuit assembly (440) may also include one or more temperature sensors. Such temperature sensors may be configured and operable like temperature sensors (364) described above. Alternatively, temperature sensors may be omitted from flex circuit assembly (440).

End effector (400) of the present example further includes a pair of position sensors (470). One position sensor (470) is positioned on proximal portion (424) of wire (420) while the other position sensor (470) is positioned on proximal portion (434) of wire (430). In addition, or in the alternative, one or more position sensors (470) may be positioned along longitudinally extending portions (426, 436), along distal portions (428, 438), on substrate (422), and/or elsewhere on end effector (400). Position sensors (470) may be configured and operable like position sensors (360) described above, such that position sensors (470) may be operable to generate signals that are indicative of the position and orientation of the corresponding portion of end effector (400) within the patient (PA). Other components and techniques may be used to generate real-time position data associated with end effector (400), in addition to or in lieu of utilizing position sensors (470).

Substrate (442) of the present example further includes a tail portion (443). Tail portion (443) of substrate (442) is adjacent to tail portions (422, 432) of wires (420, 430) and is configured to fit in or on the distal end of shaft (122). In some versions, tail portion (443) includes a plurality of termination pads (not shown) that are in electrical communication with electrodes (470) via corresponding traces (not shown) formed in flex circuit assembly (440). Wires (not shown) may be coupled with such termination pads and extend along the interior of catheter (120) to provide a path for electrical communication from electrodes (470) to console (12). In some other versions, a flex circuit extends along the length of catheter (120) to provide a path for electrical communication from electrodes (470) to console (12). For instance, tail portion (443) may extend along the length of catheter (120) and include traces that provide a path for electrical communication from electrodes (470) to console (12).

In some versions, the other side of substrate (442) includes additional electrodes that are arranged, configured, and operable just like electrodes (472) shown on the side of substrate (442) depicted in FIG. 6. In some other versions, end effector (400) includes an additional flex circuit assembly (440) that is just like flex circuit assembly (440) on the other side of end effector (400). In other words, end effector (400) may have a structural arrangement of resilient frame assembly (410) and two flex circuit assemblies (440) similar to the arrangement of end effector (300) shown in FIG. 5. Regardless of whether electrodes (472) are presented on both sides of end effector (400) on a shared single substrate (442) or on two separate substrates (442) in two separate flex circuit assemblies (440), electrodes (472) on one side of end effector (400) may contact tissue and pick up electrocardiogram signals from the tissue; while electrodes (472) on the other side of end effector (400) may contact blood that is circulating through the cardiovascular system of the patient (PA) and thereby serve as reference electrodes, picking up reference potentials from the blood to reduce noise or far field signals, as is known in the art.

While only EP mapping electrodes (472) are shown on end effector (400) in the present example, other versions of end effector (400) may include one or more ablation electrodes in addition to, or in lieu of, including EP mapping electrodes (472). Such ablation electrodes may be used to apply electrical energy to tissue that is in contact with the ablation electrodes, to thereby ablate the tissue. Each ablation electrode may be coupled with a corresponding trace or other electrical conduit of end effector (400), thereby enabling console (12) to communicate electrical energy through electrical conduits (not shown) in catheter (120) to the traces or other conduits of end effector (400) to reach the ablation electrodes. Such electrical energy may include radiofrequency (AC type) electrical energy, pulsed field (DC type) electrical energy (e.g., irreversible electroporation, etc.), or some other form of electrical energy. Alternatively, ablation electrodes may be omitted from end effector (400). As yet another variation, some versions of electrodes (472) may be configured to alternative between functionalities, including an EP mapping functionality and an ablation functionality. In some versions where electrodes (472) provide ablation functionality in addition to providing EP mapping functionality, more than two electrodes (472) may be activated simultaneously to provide ablation, such that electrodes (472) may be activated in groups to collectively provide an equivalent to an ablation electrode that is larger in size than one single electrode (472) or pair of electrodes (472).

FIG. 7 shows an end effector (500) that includes a resilient frame assembly (510) and a flex circuit assembly (540). Except as otherwise described below, end effector (500) may be configured and operable just like end effector (300) described above. End effector (500) is thus deformable to fit entirely within outer sheath (124) when outer sheath (124) is distally positioned in relation to shaft (122). End effector (500) is also configured to achieve an expanded configuration when outer sheath (124) is proximally positioned in relation to shaft (122). In the expanded configuration, end effector (500) is substantially flat such that end effector (500) extends along a substantially flat plane along which the longitudinal axis (LA) of catheter (120) extends. End effector (500) is symmetric about the longitudinal axis (LA) of catheter (120) in this example. In some other versions, end effector (500) is asymmetric about the longitudinal axis (LA) of catheter (120).

Resilient frame assembly (510) includes a first wire (520) and a second wire (530). Wires (520, 520) are configured to resiliently bias end effector (500) to the flat, expanded configuration shown in FIG. 7; while still allowing end effector (500) to compress to fit within outer sheath (124). In the present example, each wire (520, 530) is formed of nitinol, though any other suitable kind(s) of material(s) may be used to form each wire (520, 530). First wire (520) includes a tail portion (522), a proximal portion (524), a longitudinally extending portion (526), and a distal portion (528). Second wire (530) includes a tail portion (532), a proximal portion (534), a longitudinally extending portion (536), and a distal portion (538). Distal portions (528, 538) of wires (520, 430) are joined together by a union sleeve (512). In some other versions, resilient frame assembly (510) is formed by a single piece of wire such that union sleeve (512) is omitted. Tail portions (522, 432) of wires (520, 430) are configured to be secured to the distal end of shaft (122), such that end effector (500) is structurally secured to shaft (122) via tail portions (522, 532). Alternatively, any other suitable structures may be used to secure end effector (500) to shaft (122).

In the present example, proximal portions (524, 534) are oriented obliquely relative to the longitudinal axis (LA). This oblique orientation may promote collapse of end effector (500) when outer sheath (124) is advanced distally over end effector (500). In other words, the oblique orientation of proximal portions (524, 534) may promote a smooth transition for end effector (500) from the deployed, expanded state (e.g., similar to what is shown in FIG. 2B) to the contained, contracted state (e.g., similar to what is shown in FIG. 2A).

Flex circuit assembly (540) includes a substrate (542) that defines a proximal pair of obliquely extending portions (544), an inner pair of longitudinally extending portions (546), an intermediate pair of longitudinally extending portions (548), and an outer pair of longitudinally extending portions (550). Obliquely extending portions (544) diverge distally away from the longitudinal axis (LA) and form oblique angles with the longitudinal axis (LA). Longitudinally extending portions (546, 548, 550) extend between obliquely extending portions (544) of substrate (542) and distal portions (528, 538) of wires (520, 430). Each longitudinally extending portion (546, 548, 550) has one or more regions that curve laterally toward or away from the longitudinal axis (LA), such that at least a portion of each longitudinally extending portion (546, 448, 450) has a serpentine configuration. In the present example, inner longitudinally extending portions (546) are not joined together at a junction.

Substrate (542) is secured to resilient frame assembly (510) via attachment points (560, 562, 564, 566). Attachment points (560) are located at distal ends of obliquely extending portions (544). Attachment points (562) are located at distal ends of outer longitudinally extending portions (550). Attachment points (564) are located at distal ends of intermediate longitudinally extending portions (548). Attachment points (566) are located at distal ends of inner longitudinally extending portions (546). By way of example only, substrate (542) may be secured to resilient frame assembly (510) at attachment points (560, 562, 564, 566) via adhesive or epoxy, via welding, or using any other suitable kinds of components or techniques.

Flex circuit assembly (540) of the present example further includes a plurality of electrodes (572). Electrodes (572) are arranged in pairs along longitudinally extending portions (546, 548, 550) in this example. In some versions, electrodes (572) are also positioned along obliquely extending portions (544). Electrodes (572) may be configured and operable just like electrodes (362) described above, such that each pair of electrodes (572) may be operable to provide bipolar EP mapping by picking up electrocardiogram signals from tissue as is known in the art. Similarly, electrodes (572) may be provided on substrate (542) using any of the techniques described above in the context of electrodes (362) and substrate (342).

In the present example, substrate (542) is formed of a material that is flexible yet non-extensible. Substrate (542) may be formed of polyimide, polyether ether ketone, or any other suitable flex circuit substrate material(s); with electrodes (572) and associated conductive traces being printed on substrate (542). In some versions, substrate (542) is formed of a resilient material such as nitinol to assist resilient frame assembly (510) in resiliently biasing end effector (500) to the expanded configuration shown in FIG. 7 when end effector (500) is exposed relative to outer sheath (124). As another variation, substrate (542) may include a polymeric layer laid over a resilient (e.g., nitinol) layer.

It should be understood that, with at least a portion of each longitudinally extending portion (546, 548, 550) having a serpentine configuration as described above, this configuration may allow longitudinally extending portions (546, 548, 550) to deform to a greater degree than longitudinally extending portions (546, 548, 550) might otherwise deform in versions where longitudinally extending portions (546, 548, 550) would be straight. In other words, the serpentine structural aspects of longitudinally extending portions (546, 548, 550) enhance the deformability of longitudinally extending portions (546, 548, 550). As noted above, inner longitudinally extending portions (546) are not joined together at a j unction in this example. Thus, inner longitudinally extending portions (546) may have greater freedom to deform and otherwise move relative to each other as compared to the freedom of independent movement provided by inner longitudinally extending portions (446) of end effector (400). This further enhanced deformability may still further enhance the ability of electrodes (572) to reach tissue in different geometries of anatomical structures. Thus, the combination of flexible material forming substrate (542) and the serpentine structural aspects of longitudinally extending portions (546, 548, 550) may promote maximum contact between electrodes (572) and different geometries of anatomical structures.

While not shown in FIG. 7, some versions of flex circuit assembly (540) may also include one or more temperature sensors. Such temperature sensors may be configured and operable like temperature sensors (364) described above. Alternatively, temperature sensors may be omitted from flex circuit assembly (540).

End effector (500) of the present example further includes a pair of position sensors (570). One position sensor (570) is positioned on proximal portion (524) of wire (520) while the other position sensor (570) is positioned on proximal portion (534) of wire (530). In addition, or in the alternative, one or more position sensors (570) may be positioned along longitudinally extending portions (526, 536), along distal portions (528, 538), on substrate (522), and/or elsewhere on end effector (500). Position sensors (570) may be configured and operable like position sensors (360) described above, such that position sensors (570) may be operable to generate signals that are indicative of the position and orientation of the corresponding portion of end effector (500) within the patient (PA). Other components and techniques may be used to generate real-time position data associated with end effector (500), in addition to or in lieu of utilizing position sensors (570).

Substrate (542) of the present example further includes a tail portion (543). Tail portion (543) of substrate (542) is adjacent to tail portions (522, 532) of wires (520, 530) and is configured to fit in or on the distal end of shaft (122). In some versions, tail portion (543) includes a plurality of termination pads (not shown) that are in electrical communication with electrodes (470) via corresponding traces (not shown) formed in flex circuit assembly (540). Wires (not shown) may be coupled with such termination pads and extend along the interior of catheter (120) to provide a path for electrical communication from electrodes (570) to console (12). In some other versions, a flex circuit extends along the length of catheter (120) to provide a path for electrical communication from electrodes (570) to console (12). For instance, tail portion (543) may extend along the length of catheter (120) and include traces that provide a path for electrical communication from electrodes (570) to console (12).

In some versions, the other side of substrate (542) includes additional electrodes that are arranged, configured, and operable just like electrodes (572) shown on the side of substrate (542) depicted in FIG. 7. In some other versions, end effector (500) includes an additional flex circuit assembly (540) that is just like flex circuit assembly (540) on the other side of end effector (500). In other words, end effector (500) may have a structural arrangement of resilient frame assembly (510) and two flex circuit assemblies (540) similar to the arrangement of end effector (300) shown in FIG. 5. Regardless of whether electrodes (572) are presented on both sides of end effector (500) on a shared single substrate (542) or on two separate substrates (542) in two separate flex circuit assemblies (540), electrodes (572) on one side of end effector (500) may contact tissue and pick up electrocardiogram signals from the tissue; while electrodes (572) on the other side of end effector (500) may contact blood that is circulating through the cardiovascular system of the patient (PA) and thereby serve as reference electrodes, picking up reference potentials from the blood to reduce noise or far field signals, as is known in the art.

While only EP mapping electrodes (572) are shown on end effector (500) in the present example, other versions of end effector (500) may include one or more ablation electrodes in addition to, or in lieu of, including EP mapping electrodes (572). Such ablation electrodes may be used to apply electrical energy to tissue that is in contact with the ablation electrodes, to thereby ablate the tissue. Each ablation electrode may be coupled with a corresponding trace or other electrical conduit of end effector (500), thereby enabling console (12) to communicate electrical energy through electrical conduits (not shown) in catheter (120) to the traces or other conduits of end effector (500) to reach the ablation electrodes. Such electrical energy may include radiofrequency (AC type) electrical energy, pulsed field (DC type) electrical energy (e.g., irreversible electroporation, etc.), or some other form of electrical energy. Alternatively, ablation electrodes may be omitted from end effector (500). As yet another variation, some versions of electrodes (572) may be configured to alternative between functionalities, including an EP mapping functionality and an ablation functionality. In some versions where electrodes (572) provide ablation functionality in addition to providing EP mapping functionality, more than two electrodes (572) may be activated simultaneously to provide ablation, such that electrodes (572) may be activated in groups to collectively provide an equivalent to an ablation electrode that is larger in size than one single electrode (572) or pair of electrodes (572).

### C. End Effector with Resilient Frame and Extensible Flexible Interior

As noted above in the description of end effectors (400, 500), a flex circuit may provide substantial flexibility despite having a non-extensible substrate, particularly when the substrate has serpentine structural features. In some scenarios, it may be desirable for a flex circuit of an end effector to provide extensible flexibility. In some such scenarios, the flex circuit substrate need not necessarily incorporate serpentine structural features (though extensible flex circuit substrates may in fact incorporate serpentine structural features, if desired). In any case, an end effector having a flex circuit with an extensible substrate may provide a desired degree of flexibility to promote conformance with the different geometries of different anatomical structures within a cardiovascular system. Examples of end effectors providing such functionality are described in greater detail below.

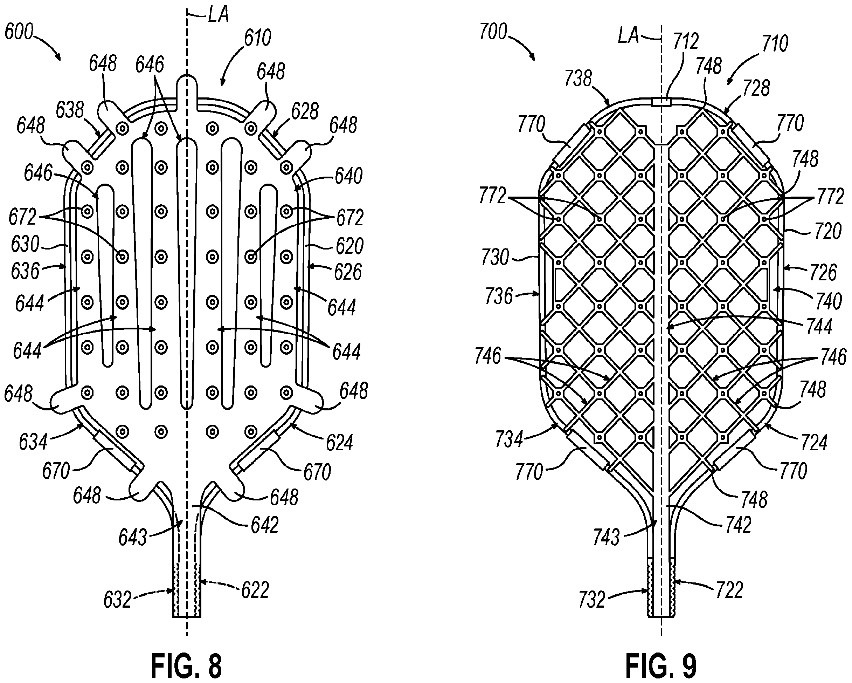

FIG. 8 shows an end effector (600) that includes a resilient frame assembly (610) and a flex circuit assembly (640). Except as otherwise described below, end effector (600) may be configured and operable just like end effector (300) described above. End effector (600) is thus deformable to fit entirely within outer sheath (124) when outer sheath (124) is distally positioned in relation to shaft (122). End effector (600) is also configured to achieve an expanded configuration when outer sheath (124) is proximally positioned in relation to shaft (122). In the expanded configuration, end effector (600) is substantially flat such that end effector (600) extends along a substantially flat plane along which the longitudinal axis (LA) of catheter (120) extends. End effector (600) is symmetric about the longitudinal axis (LA) of catheter (120) in this example. In some other versions, end effector (600) is asymmetric about the longitudinal axis (LA) of catheter (120).

Resilient frame assembly (610) includes a first wire (620) and a second wire (630). Wires (620, 630) are configured to resiliently bias end effector (600) to the flat, expanded configuration shown in FIG. 8; while still allowing end effector (600) to compress to fit within outer sheath (124). In the present example, each wire (620, 630) is formed of nitinol, though any other suitable kind(s) of material(s) may be used to form each wire (620, 630). First wire (620) includes a tail portion (622), a proximal portion (624), a longitudinally extending portion (626), and a distal portion (628). Second wire (630) includes a tail portion (632), a proximal portion (634), a longitudinally extending portion (636), and a distal portion (638). Distal portions (628, 638) of wires (620, 630) are joined together by a union sleeve, which is not shown in FIG. 8 but may be configured like any of the other union sleeves shown in any of the other drawings. In some other versions, resilient frame assembly (610) is formed by a single piece of wire such that the union sleeve is omitted. Tail portions (622, 632) of wires (620, 630) are configured to be secured to the distal end of shaft (122), such that end effector (600) is structurally secured to shaft (122) via tail portions (622, 632). Alternatively, any other suitable structures may be used to secure end effector (600) to shaft (122).

In the present example, proximal portions (624, 634) are oriented obliquely relative to the longitudinal axis (LA). This oblique orientation may promote collapse of end effector (600) when outer sheath (124) is advanced distally over end effector (600). In other words, the oblique orientation of proximal portions (624, 634) may promote a smooth transition for end effector (600) from the deployed, expanded state (e.g., similar to what is shown in FIG. 2B) to the contained, contracted state (e.g., similar to what is shown in FIG. 2A).

Flex circuit assembly (640) includes a substrate (642) that defines a plurality of longitudinally extending portions (644) a plurality of connection tabs (648), and a tail portion (643). Longitudinally extending portions (644) define a plurality of elongate openings (646). Substrate (642) is secured to resilient frame assembly (610) via connection tabs (648). In some versions, connection tabs (648) are wrapped about wires (620, 630) and fixedly secured in the wrapped state, thereby securing substrate (642) to resilient frame assembly (610). In some other versions, connection tabs (648) of a flex circuit assembly (640) on one side of end effector (600) are secured to connection tabs (648) of another flex circuit assembly (640) on the other side of end effector (600), such that the two flex circuit assemblies (640) are secured together and thereby effectively secure each other relative to resilient frame assembly (610). Alternatively, substrate (642) may be secured to resilient frame assembly (610) in any other suitable fashion.

Flex circuit assembly (640) of the present example further includes a plurality of electrodes (672). Electrodes (672) are arranged in pairs along substrate (642) in this example. Electrodes (672) may be configured and operable just like electrodes (362) described above, such that each pair of electrodes (672) may be operable to provide bipolar EP mapping by picking up electrocardiogram signals from tissue as is known in the art. Similarly, electrodes (672) may be provided on substrate (642) using any of the techniques described above in the context of electrodes (362) and substrate (342).

In the present example, substrate (642) is formed of a material that is flexible and extensible. Substrate (642) may be formed of silicone, nitinol, or any other suitable extensible flexible substrate material(s); with electrodes (672) and associated conductive traces being printed on substrate (642) or otherwise integrated with substrate (642). In some versions, a resilient material may be integrated into substrate (642) to further assist resilient frame assembly (610) in resiliently biasing end effector (600) to the expanded configuration shown in FIG. 8 when end effector (600) is exposed relative to outer sheath (124). In such versions, the resilient material may be configured and positioned to allow substrate (642) to stretch without damaging substrate (642).

It should be understood that, with substrate (642) being formed of an extensible material, this extensibility may allow substrate (642) to deform to a greater degree than substrate (642) might otherwise deform in versions where substrate (642) is non-extensible. In other words, the extensibility of substrate (642) may further enhance the ability of electrodes (672) to reach tissue in different geometries of anatomical structures. Thus, the extensibility of substrate (642) may promote maximum contact between electrodes (672) and different geometries of anatomical structures.

With substrate (642) being extensible in the present example, it may be desirable to provide traces, wires, or other electrical conduits in substrate (642) that are configured to accommodate stretching of substrate (642) while still providing electrical continuity to electrodes (672) and any other electrical components in end effector (600). To that end, it may be desirable for such traces, wires, or other electrical conduits in substrate (642) to have a serpentine configuration, zig-zag configuration, or other kind of structural configuration that allows substrate (642) to stretch while still providing electrical continuity to electrodes (672) and any other electrical components in end effector (600). Thus, even if the traces, wires, or other electrical conduits in substrate (642) are not themselves formed of extensible material, the structural configuration of the traces, wires, or other electrical conduits in substrate (642) may nevertheless allow such traces, wires, or other electrical conduits in substrate (642) to deform as substrate (642) as stretches while still providing electrical continuity to electrodes (672) and any other electrical components in end effector (600).

While not shown in FIG. 8, some versions of flex circuit assembly (640) may also include one or more temperature sensors. Such temperature sensors may be configured and operable like temperature sensors (364) described above. Alternatively, temperature sensors may be omitted from flex circuit assembly (640).

End effector (600) of the present example further includes a pair of position sensors (670). One position sensor (670) is positioned on proximal portion (624) of wire (620) while the other position sensor (670) is positioned on proximal portion (634) of wire (630). In addition, or in the alternative, one or more position sensors (670) may be positioned along longitudinally extending portions (626, 636), along distal portions (628, 638), on substrate (622), and/or elsewhere on end effector (600). Position sensors (670) may be configured and operable like position sensors (360) described above, such that position sensors (670) may be operable to generate signals that are indicative of the position and orientation of the corresponding portion of end effector (600) within the patient (PA). Other components and techniques may be used to generate real-time position data associated with end effector (600), in addition to or in lieu of utilizing position sensors (670).

Substrate (642) of the present example further includes a tail portion (643). Tail portion (643) of substrate (642) is adjacent to tail portions (622, 632) of wires (620, 630) and is configured to fit in or on the distal end of shaft (122). In some versions, tail portion (643) includes a plurality of termination pads (not shown) that are in electrical communication with electrodes (670) via corresponding traces (not shown) formed in flex circuit assembly (640). Wires (not shown) may be coupled with such termination pads and extend along the interior of catheter (120) to provide a path for electrical communication from electrodes (670) to console (12). In some other versions, a flex circuit extends along the length of catheter (120) to provide a path for electrical communication from electrodes (670) to console (12). For instance, tail portion (643) may extend along the length of catheter (120) and include traces that provide a path for electrical communication from electrodes (670) to console (12).

In some versions, the other side of substrate (642) includes additional electrodes that are arranged, configured, and operable just like electrodes (672) shown on the side of substrate (642) depicted in FIG. 8. In some other versions, end effector (600) includes an additional flex circuit assembly (640) that is just like flex circuit assembly (640) on the other side of end effector (600). In other words, end effector (600) may have a structural arrangement of resilient frame assembly (610) and two flex circuit assemblies (640) similar to the arrangement of end effector (300) shown in FIG. 5. Regardless of whether electrodes (672) are presented on both sides of end effector (600) on a shared single substrate (642) or on two separate substrates (642) in two separate flex circuit assemblies (640), electrodes (672) on one side of end effector (600) may contact tissue and pick up electrocardiogram signals from the tissue; while electrodes (672) on the other side of end effector (600) may contact blood that is circulating through the cardiovascular system of the patient (PA) and thereby serve as reference electrodes, picking up reference potentials from the blood to reduce noise or far field signals, as is known in the art.

While only EP mapping electrodes (672) are shown on end effector (600) in the present example, other versions of end effector (600) may include one or more ablation electrodes in addition to, or in lieu of, including EP mapping electrodes (672). Such ablation electrodes may be used to apply electrical energy to tissue that is in contact with the ablation electrodes, to thereby ablate the tissue. Each ablation electrode may be coupled with a corresponding trace or other electrical conduit of end effector (600), thereby enabling console (12) to communicate electrical energy through electrical conduits (not shown) in catheter (120) to the traces or other conduits of end effector (600) to reach the ablation electrodes. Such electrical energy may include radiofrequency (AC type) electrical energy, pulsed field (DC type) electrical energy (e.g., irreversible electroporation, etc.), or some other form of electrical energy. Alternatively, ablation electrodes may be omitted from end effector (600). As yet another variation, some versions of electrodes (672) may be configured to alternative between functionalities, including an EP mapping functionality and an ablation functionality. In some versions where electrodes (672) provide ablation functionality in addition to providing EP mapping functionality, more than two electrodes (672) may be activated simultaneously to provide ablation, such that electrodes (672) may be activated in groups to collectively provide an equivalent to an ablation electrode that is larger in size than one single electrode (672) or pair of electrodes (672).

FIG. 9 shows an end effector (700) that includes a resilient frame assembly (710) and a flex circuit assembly (740). Except as otherwise described below, end effector (700) may be configured and operable just like end effector (300) described above. End effector (700) is thus deformable to fit entirely within outer sheath (124) when outer sheath (124) is distally positioned in relation to shaft (122). End effector (700) is also configured to achieve an expanded configuration when outer sheath (124) is proximally positioned in relation to shaft (122). In the expanded configuration, end effector (700) is substantially flat such that end effector (700) extends along a substantially flat plane along which the longitudinal axis (LA) of catheter (120) extends. End effector (700) is symmetric about the longitudinal axis (LA) of catheter (120) in this example. In some other versions, end effector (700) is asymmetric about the longitudinal axis (LA) of catheter (120).

Resilient frame assembly (710) includes a first wire (720) and a second wire (730). Wires (720, 730) are configured to resiliently bias end effector (700) to the flat, expanded configuration shown in FIG. 9; while still allowing end effector (700) to compress to fit within outer sheath (124). In the present example, each wire (720, 730) is formed of nitinol, though any other suitable kind(s) of material(s) may be used to form each wire (720, 730). First wire (720) includes a tail portion (722), a proximal portion (724), a longitudinally extending portion (726), and a distal portion (728). Second wire (730) includes a tail portion (732), a proximal portion (734), a longitudinally extending portion (736), and a distal portion (738). Distal portions (728, 738) of wires (720, 730) are joined together by a union sleeve (712). In some other versions, resilient frame assembly (710) is formed by a single piece of wire such that union sleeve (712) is omitted. Tail portions (722, 732) of wires (720, 730) are configured to be secured to the distal end of shaft (122), such that end effector (700) is structurally secured to shaft (122) via tail portions (722, 732). Alternatively, any other suitable structures may be used to secure end effector (700) to shaft (122).

In the present example, proximal portions (724, 734) are oriented obliquely relative to the longitudinal axis (LA). This oblique orientation may promote collapse of end effector (700) when outer sheath (124) is advanced distally over end effector (700). In other words, the oblique orientation of proximal portions (724, 734) may promote a smooth transition for end effector (700) from the deployed, expanded state (e.g., similar to what is shown in FIG. 2B) to the contained, contracted state (e.g., similar to what is shown in FIG. 2A).

Flex circuit assembly (740) includes a substrate (742) that defines a central longitudinally extending portion (744), a plurality of web portions (746), and a tail portion (743). Web portions (746) extend obliquely from longitudinally extending portion (744), which extends along the longitudinal axis (LA). Web portions (746) cooperate to define a grid configuration, with the grid being rotated askew relative to the longitudinal axis (LA). Substrate (742) is secured to resilient frame assembly (710) via attachment points (748). Attachment points (748) are located at ends of web portions (746). In some versions, attachment points (748) are secured directly to resilient frame assembly (710) via adhesive or epoxy, via welding, or using any other suitable kinds of components or techniques. In some other versions, attachment points (748) represent regions of web portions (746) that wrap around resilient frame assembly (710) to join with corresponding regions of web portions (746) of another flex circuit assembly (740) that is on the other side of end effector (700). In such versions, the two flex circuit assemblies (740) are secured together and thereby effectively secure each other relative to resilient frame assembly (710). Alternatively, substrate (742) may be secured to resilient frame assembly (710) in any other suitable fashion.

Flex circuit assembly (740) of the present example further includes a plurality of electrodes (772). Electrodes (772) are arranged in pairs along substrate (742) in this example, at intersections of web portions (746). Electrodes (772) may be configured and operable just like electrodes (362) described above, such that each pair of electrodes (772) may be operable to provide bipolar EP mapping by picking up electrocardiogram signals from tissue as is known in the art. Similarly, electrodes (772) may be provided on substrate (742) using any of the techniques described above in the context of electrodes (362) and substrate (342).

In the present example, substrate (742) is formed of a material that is flexible and extensible. Substrate (742) may be formed of silicone, nitinol, or any other suitable extensible flexible substrate material(s); with electrodes (772) and associated conductive traces being printed on substrate (742) or otherwise integrated with substrate (742). In some versions, a resilient material may be integrated into substrate (742) to further assist resilient frame assembly (710) in resiliently biasing end effector (700) to the expanded configuration shown in FIG. 9 when end effector (700) is exposed relative to outer sheath (124). In such versions, the resilient material may be configured and positioned to allow substrate (742) to stretch without damaging substrate (742).

It should be understood that, with substrate (742) being formed of an extensible material, this extensibility may allow substrate (742) to deform to a greater degree than substrate (742) might otherwise deform in versions where substrate (742) is non-extensible. Moreover, the grid configuration provided by substrate (742) may also enhance the extensibility of substrate (742) (e.g., as compared to the extensibility of substrate (642). In other words, the extensibility of substrate (742) and the grid configuration of substrate (742) may further enhance the ability of electrodes (772) to reach tissue in different geometries of anatomical structures. Thus, the extensibility of substrate (742) and the grid configuration of substrate (742) may promote maximum contact between electrodes (772) and different geometries of anatomical structures.

With substrate (742) being extensible in the present example, it may be desirable to provide traces, wires, or other electrical conduits in substrate (742) that are configured to accommodate stretching of substrate (742) while still providing electrical continuity to electrodes (772) and any other electrical components in end effector (700). To that end, it may be desirable for such traces, wires, or other electrical conduits in substrate (742) to have a serpentine configuration, zig-zag configuration, or other kind of structural configuration that allows substrate (742) to stretch while still providing electrical continuity to electrodes (772) and any other electrical components in end effector (700). Thus, even if the traces, wires, or other electrical conduits in substrate (742) are not themselves formed of extensible material, the structural configuration of the traces, wires, or other electrical conduits in substrate (742) may nevertheless allow such traces, wires, or other electrical conduits in substrate (742) to deform as substrate (742) as stretches while still providing electrical continuity to electrodes (772) and any other electrical components in end effector (700).

While not shown in FIG. 9, some versions of flex circuit assembly (740) may also include one or more temperature sensors. Such temperature sensors may be configured and operable like temperature sensors (364) described above. Alternatively, temperature sensors may be omitted from flex circuit assembly (740).

End effector (700) of the present example further includes a pair of position sensors (770). One position sensor (770) is positioned on proximal portion (724) of wire (720) while the other position sensor (770) is positioned on proximal portion (734) of wire (730). In addition, or in the alternative, one or more position sensors (770) may be positioned along longitudinally extending portions (726, 736), along distal portions (728, 738), on substrate (722), and/or elsewhere on end effector (700). Position sensors (770) may be configured and operable like position sensors (360) described above, such that position sensors (770) may be operable to generate signals that are indicative of the position and orientation of the corresponding portion of end effector (700) within the patient (PA). Other components and techniques may be used to generate real-time position data associated with end effector (700), in addition to or in lieu of utilizing position sensors (770).

Substrate (742) of the present example further includes a tail portion (743). Tail portion (743) of substrate (742) is adjacent to tail portions (722, 732) of wires (720, 730) and is configured to fit in or on the distal end of shaft (122). In some versions, tail portion (743) includes a plurality of termination pads (not shown) that are in electrical communication with electrodes (770) via corresponding traces (not shown) formed in flex circuit assembly (740). Wires (not shown) may be coupled with such termination pads and extend along the interior of catheter (120) to provide a path for electrical communication from electrodes (770) to console (12). In some other versions, a flex circuit extends along the length of catheter (120) to provide a path for electrical communication from electrodes (770) to console (12). For instance, tail portion (743) may extend along the length of catheter (120) and include traces that provide a path for electrical communication from electrodes (770) to console (12).

In some versions, the other side of substrate (742) includes additional electrodes that are arranged, configured, and operable just like electrodes (772) shown on the side of substrate (742) depicted in FIG. 9. In some other versions, end effector (700) includes an additional flex circuit assembly (740) that is just like flex circuit assembly (740) on the other side of end effector (700). In other words, end effector (700) may have a structural arrangement of resilient frame assembly (710) and two flex circuit assemblies (740) similar to the arrangement of end effector (300) shown in FIG. 5. Regardless of whether electrodes (772) are presented on both sides of end effector (700) on a shared single substrate (742) or on two separate substrates (742) in two separate flex circuit assemblies (740), electrodes (772) on one side of end effector (700) may contact tissue and pick up electrocardiogram signals from the tissue; while electrodes (772) on the other side of end effector (700) may contact blood that is circulating through the cardiovascular system of the patient (PA) and thereby serve as reference electrodes, picking up reference potentials from the blood to reduce noise or far field signals, as is known in the art.

While only EP mapping electrodes (772) are shown on end effector (700) in the present example, other versions of end effector (700) may include one or more ablation electrodes in addition to, or in lieu of, including EP mapping electrodes (772). Such ablation electrodes may be used to apply electrical energy to tissue that is in contact with the ablation electrodes, to thereby ablate the tissue. Each ablation electrode may be coupled with a corresponding trace or other electrical conduit of end effector (700), thereby enabling console (12) to communicate electrical energy through electrical conduits (not shown) in catheter (120) to the traces or other conduits of end effector (700) to reach the ablation electrodes. Such electrical energy may include radiofrequency (AC type) electrical energy, pulsed field (DC type) electrical energy (e.g., irreversible electroporation, etc.), or some other form of electrical energy. Alternatively, ablation electrodes may be omitted from end effector (700). As yet another variation, some versions of electrodes (772) may be configured to alternative between functionalities, including an EP mapping functionality and an ablation functionality. In some versions where electrodes (772) provide ablation functionality in addition to providing EP mapping functionality, more than two electrodes (772) may be activated simultaneously to provide ablation, such that electrodes (772) may be activated in groups to collectively provide an equivalent to an ablation electrode that is larger in size than one single electrode (772) or pair of electrodes (772).

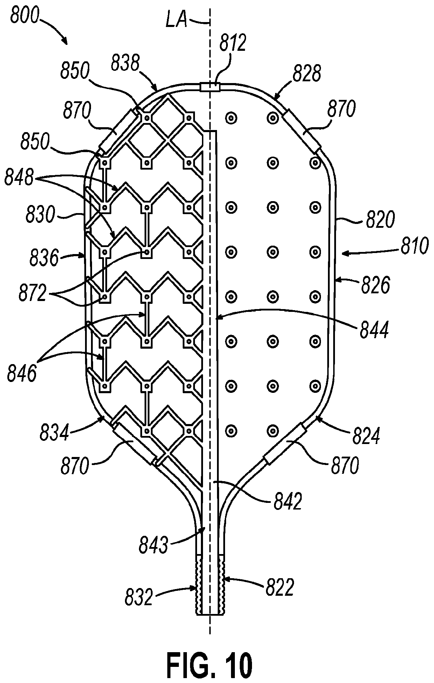

FIG. 10 shows an end effector (800) that includes a resilient frame assembly (810) and a flex circuit assembly (840). Except as otherwise described below, end effector (800) may be configured and operable just like end effector (300) described above. End effector (800) is thus deformable to fit entirely within outer sheath (124) when outer sheath (124) is distally positioned in relation to shaft (122). End effector (800) is also configured to achieve an expanded configuration when outer sheath (124) is proximally positioned in relation to shaft (122). In the expanded configuration, end effector (800) is substantially flat such that end effector (800) extends along a substantially flat plane along which the longitudinal axis (LA) of catheter (120) extends. End effector (800) is symmetric about the longitudinal axis (LA) of catheter (120) in this example. In some other versions, end effector (800) is asymmetric about the longitudinal axis (LA) of catheter (120).

Resilient frame assembly (810) includes a first wire (820) and a second wire (830). Wires (820, 830) are configured to resiliently bias end effector (800) to the flat, expanded configuration shown in FIG. 10; while still allowing end effector (800) to compress to fit within outer sheath (124). In the present example, each wire (820, 830) is formed of nitinol, though any other suitable kind(s) of material(s) may be used to form each wire (820, 830). First wire (820) includes a tail portion (822), a proximal portion (824), a longitudinally extending portion (826), and a distal portion (828). Second wire (830) includes a tail portion (832), a proximal portion (834), a longitudinally extending portion (836), and a distal portion (838). Distal portions (828, 838) of wires (820, 830) are joined together by a union sleeve (812). In some other versions, resilient frame assembly (610) is formed by a single piece of wire such that union sleeve (812) is omitted. Tail portions (822, 832) of wires (820, 830) are configured to be secured to the distal end of shaft (122), such that end effector (800) is structurally secured to shaft (122) via tail portions (822, 832). Alternatively, any other suitable structures may be used to secure end effector (800) to shaft (122).

In the present example, proximal portions (824, 834) are oriented obliquely relative to the longitudinal axis (LA). This oblique orientation may promote collapse of end effector (800) when outer sheath (124) is advanced distally over end effector (800). In other words, the oblique orientation of proximal portions (824, 834) may promote a smooth transition for end effector (800) from the deployed, expanded state (e.g., similar to what is shown in FIG. 2B) to the contained, contracted state (e.g., similar to what is shown in FIG. 2A).

Flex circuit assembly (840) includes a substrate (842) that defines a central longitudinally extending portion (844), a plurality of longitudinally extending segments (846), a plurality of zig-zag segments (848), and a tail portion (843). While FIG. 10 only shows longitudinally extending segments (846) and zig-zag segments (848) on one side of the longitudinal axis (LA), it should be understood that substrate (842) may be symmetric about the longitudinal axis (LA) such that longitudinally extending segments (846) and zig-zag segments (848) may also be positioned on the other side of the longitudinal axis (LA). Longitudinally extending segments (846) are laterally offset from central longitudinally extending portion (844) and are longitudinally offset relative to each other. Zig-zag segments (848) extend transversely from central longitudinally extending portion (844). Longitudinally extending segments (846) extend between zig-zag segments (848). While zig-zag segments (848) have a zig-zag configuration in the present example, segments (848) may have a serpentine configuration or other kind of configuration in other versions.

Substrate (842) is secured to resilient frame assembly (810) via attachment points (850). Attachment points (850) are located at ends of zig-zag segments (846). In some versions, attachment points (850) are secured directly to resilient frame assembly (810) via adhesive or epoxy, via welding, or using any other suitable kinds of components or techniques. In some other versions, attachment points (850) represent regions of zig-zag segments (848) that wrap around resilient frame assembly (810) to join with corresponding regions of zig-zag segments (848) of another flex circuit assembly (840) that is on the other side of end effector (800). In such versions, the two flex circuit assemblies (840) are secured together and thereby effectively secure each other relative to resilient frame assembly (810). Alternatively, substrate (842) may be secured to resilient frame assembly (810) in any other suitable fashion.

Flex circuit assembly (840) of the present example further includes a plurality of electrodes (872). Electrodes (872) are arranged in pairs along substrate (842) in this example, at intersections of zig-zag segments (848) and longitudinally extending segments (846); and at regions of zig-zag segments (848) next to central longitudinally extending portion (844). Electrodes (872) may be configured and operable just like electrodes (362) described above, such that each pair of electrodes (872) may be operable to provide bipolar EP mapping by picking up electrocardiogram signals from tissue as is known in the art. Similarly, electrodes (872) may be provided on substrate (842) using any of the techniques described above in the context of electrodes (362) and substrate (342).

In the present example, substrate (842) is formed of a material that is flexible and extensible. Substrate (842) may be formed of silicone, nitinol, or any other suitable extensible flexible substrate material(s); with electrodes (872) and associated conductive traces being printed on substrate (842) or otherwise integrated with substrate (842). In some versions, a resilient material may be integrated into substrate (842) to further assist resilient frame assembly (810) in resiliently biasing end effector (800) to the expanded configuration shown in FIG. 10 when end effector (800) is exposed relative to outer sheath (124). In such versions, the resilient material may be configured and positioned to allow substrate (842) to stretch without damaging substrate (842).

It should be understood that, with substrate (842) being formed of an extensible material, this extensibility may allow substrate (842) to deform to a greater degree than substrate (842) might otherwise deform in versions where substrate (842) is non-extensible. Moreover, the zig-zag configuration provided by zig-zag segments (848) may also enhance the extensibility of substrate (842) (e.g., as compared to the extensibility of substrate (642). In other words, the extensibility of substrate (842) and the zig-zag configuration provided by zig-zag segments (848) may further enhance the ability of electrodes (872) to reach tissue in different geometries of anatomical structures. Thus, the extensibility of substrate (842) and the zig-zag configuration provided by zig-zag segments (848) may promote maximum contact between electrodes (872) and different geometries of anatomical structures.

With substrate (842) being extensible in the present example, it may be desirable to provide traces, wires, or other electrical conduits in substrate (842) that are configured to accommodate stretching of substrate (842) while still providing electrical continuity to electrodes (872) and any other electrical components in end effector (800). To that end, it may be desirable for such traces, wires, or other electrical conduits in substrate (842) to have a serpentine configuration, zig-zag configuration, or other kind of structural configuration that allows substrate (842) to stretch while still providing electrical continuity to electrodes (872) and any other electrical components in end effector (800). Thus, even if the traces, wires, or other electrical conduits in substrate (842) are not themselves formed of extensible material, the structural configuration of the traces, wires, or other electrical conduits in substrate (842) may nevertheless allow such traces, wires, or other electrical conduits in substrate (842) to deform as substrate (842) as stretches while still providing electrical continuity to electrodes (872) and any other electrical components in end effector (800).

While not shown in FIG. 10, some versions of flex circuit assembly (840) may also include one or more temperature sensors. Such temperature sensors may be configured and operable like temperature sensors (364) described above. Alternatively, temperature sensors may be omitted from flex circuit assembly (840).

End effector (800) of the present example further includes a pair of position sensors (870). One position sensor (870) is positioned on proximal portion (824) of wire (820) while the other position sensor (870) is positioned on proximal portion (834) of wire (830). In addition, or in the alternative, one or more position sensors (870) may be positioned along longitudinally extending portions (826, 836), along distal portions (828, 838), on substrate (822), and/or elsewhere on end effector (800). Position sensors (870) may be configured and operable like position sensors (360) described above, such that position sensors (870) may be operable to generate signals that are indicative of the position and orientation of the corresponding portion of end effector (800) within the patient (PA). Other components and techniques may be used to generate real-time position data associated with end effector (800), in addition to or in lieu of utilizing position sensors (870).

Substrate (842) of the present example further includes a tail portion (843). Tail portion (843) of substrate (842) is adjacent to tail portions (822, 832) of wires (820, 830) and is configured to fit in or on the distal end of shaft (122). In some versions, tail portion (843) includes a plurality of termination pads (not shown) that are in electrical communication with electrodes (870) via corresponding traces (not shown) formed in flex circuit assembly (840). Wires (not shown) may be coupled with such termination pads and extend along the interior of catheter (120) to provide a path for electrical communication from electrodes (870) to console (12). In some other versions, a flex circuit extends along the length of catheter (120) to provide a path for electrical communication from electrodes (870) to console (12). For instance, tail portion (843) may extend along the length of catheter (120) and include traces that provide a path for electrical communication from electrodes (870) to console (12).

In some versions, the other side of substrate (842) includes additional electrodes that are arranged, configured, and operable just like electrodes (872) shown on the side of substrate (842) depicted in FIG. 10. In some other versions, end effector (800) includes an additional flex circuit assembly (840) that is just like flex circuit assembly (840) on the other side of end effector (800). In other words, end effector (800) may have a structural arrangement of resilient frame assembly (810) and two flex circuit assemblies (840) similar to the arrangement of end effector (300) shown in FIG. 5. Regardless of whether electrodes (872) are presented on both sides of end effector (800) on a shared single substrate (842) or on two separate substrates (842) in two separate flex circuit assemblies (840), electrodes (872) on one side of end effector (800) may contact tissue and pick up electrocardiogram signals from the tissue; while electrodes (872) on the other side of end effector (800) may contact blood that is circulating through the cardiovascular system of the patient (PA) and thereby serve as reference electrodes, picking up reference potentials from the blood to reduce noise or far field signals, as is known in the art.

While only EP mapping electrodes (872) are shown on end effector (800) in the present example, other versions of end effector (800) may include one or more ablation electrodes in addition to, or in lieu of, including EP mapping electrodes (872). Such ablation electrodes may be used to apply electrical energy to tissue that is in contact with the ablation electrodes, to thereby ablate the tissue. Each ablation electrode may be coupled with a corresponding trace or other electrical conduit of end effector (800), thereby enabling console (12) to communicate electrical energy through electrical conduits (not shown) in catheter (120) to the traces or other conduits of end effector (800) to reach the ablation electrodes. Such electrical energy may include radiofrequency (AC type) electrical energy, pulsed field (DC type) electrical energy (e.g., irreversible electroporation, etc.), or some other form of electrical energy. Alternatively, ablation electrodes may be omitted from end effector (800). As yet another variation, some versions of electrodes (872) may be configured to alternative between functionalities, including an EP mapping functionality and an ablation functionality. In some versions where electrodes (872) provide ablation functionality in addition to providing EP mapping functionality, more than two electrodes (872) may be activated simultaneously to provide ablation, such that electrodes (872) may be activated in groups to collectively provide an equivalent to an ablation electrode that is larger in size than one single electrode (872) or pair of electrodes (872).

### III. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus comprising: (a) a catheter shaft assembly having a proximal end and a distal end, the catheter shaft assembly defining a longitudinal axis; and (b) an end effector associated with the distal end of the catheter shaft assembly, the end effector being configured to transition between a first configuration and a second configuration, the end effector being configured to fit within an outer sheath in the first configuration, the end effector being configured to expand outwardly away from the longitudinal axis in the second configuration when exposed distally relative to the distal end of the outer sheath, the end effector comprising: (i) a resilient frame assembly, the resilient frame assembly being configured to resiliently bias the end effector toward the second configuration, and (ii) a first flex circuit assembly secured to the resilient frame assembly, the first flex circuit assembly comprising: (A) a first flexible substrate, and (B) a first plurality of electrodes positioned on the first flexible substrate, the first plurality of electrodes being configured perform one or both of: (1) picking up electrical potentials from tissue or blood, or (2) ablating tissue.

### Example 2

The apparatus of Example 1, the end effector being configured to define a flat, planar shape in the second configuration.

### Example 3

The apparatus of any of Examples 1 through 2, the resilient frame assembly comprising nitinol.

### Example 4

The apparatus of any of Examples 1 through 3, the resilient frame assembly comprising at least one resilient wire.

### Example 5

The apparatus of Example 4, the resilient frame assembly comprising a first wire and a second wire joined together.

### Example 6

The apparatus of any of Examples 1 through 5, the resilient frame assembly forming a loop shape.

### Example 7

The apparatus of any of Examples 1 through 6, the first flexible substrate comprising a resilient material.

### Example 8

The apparatus of any of Examples 1 through 7, the first flexible substrate comprising an extensible material.

### Example 9

The apparatus of any of Examples 1 through 7, the first flexible substrate comprising a non-extensible material.

### Example 10

The apparatus of any of Examples 1 through 9, the first flexible substrate comprising one or more elongation features.

### Example 11

The apparatus of Example 10, the one or more elongation features comprising one or more serpentine curves.

### Example 12

The apparatus of Example 10, the one or more elongation features comprising one or more zig-zag structures.

### Example 13

The apparatus of any of Examples 1 through 12, the first flexible substrate comprising nitinol.

### Example 14

The apparatus of any of Examples 1 through 13, the first flexible substrate comprising silicone.

### Example 15

The apparatus of any of Examples 1 through 14, the end effector further comprising a second flex circuit assembly secured to the resilient frame assembly, the second flex circuit assembly comprising: (A) a second flexible substrate, and (B) a second plurality of electrodes positioned on the second flexible substrate, the second plurality of electrodes being configured perform one or both of: (1) picking up electrical potentials from tissue or blood, or (2) ablating tissue.

### Example 16

The apparatus of Example 15, the first flex circuit assembly being positioned on a first side of the end effector, the second flex circuit assembly being positioned on a second side of the end effector, the second side being opposite to the first side.

### Example 17

The apparatus of any of Examples 1 through 16, the end effector further comprising a second plurality of electrodes, the first plurality of electrodes being positioned on a first side of the end effector, the second plurality of electrodes being positioned on a second side of the end effector, the second side being opposite to the first side.

### Example 18

The apparatus of Example 17, the end effector further comprising a second flex circuit assembly secured to the resilient frame assembly, the second flex circuit assembly comprising a second flexible substrate, the second plurality of electrodes being positioned on the second flexible substrate.

### Example 19

The apparatus of any of Examples 1 through 18, at least a portion of the first flexible substrate defining a serpentine configuration.

### Example 20

The apparatus of any of Examples 1 through 19, at least a portion of the first flexible substrate defining a grid configuration.

### Example 21

The apparatus of any of Examples 1 through 20, at least a portion of the first flexible substrate defining a zig-zag configuration.

### Example 22

The apparatus of any of Examples 1 through 21, the end effector further comprising one or more temperature sensors.

### Example 23

The apparatus of Example 22, the one or more temperature sensors being positioned on the first flexible substrate.

### Example 24

The apparatus of any of Examples 1 through 23, the end effector further comprising one or more position sensors.

### Example 25

The apparatus of Example 24, the one or more position sensors being positioned on the resilient frame assembly.

### Example 26

The apparatus of any of Examples 1 through 25, the first flexible substrate including a portion extending obliquely relative to the longitudinal axis.

### Example 27

The apparatus of any of Examples 1 through 26, the resilient frame assembly including a portion extending obliquely relative to the longitudinal axis.

### Example 28

The apparatus of any of Examples 1 through 27, the catheter shaft assembly comprising an inner shaft and the outer sheath.

### Example 29

The apparatus of Example 28, the outer sheath being longitudinally translatable relative to the inner shaft.

### Example 30

An apparatus comprising: (a) a catheter shaft assembly having a proximal end and a distal end, the catheter shaft assembly defining a longitudinal axis; and (b) an end effector associated with the distal end of the catheter shaft assembly, the end effector comprising: (i) a resilient frame assembly, the resilient frame assembly being configured to resiliently bias the end effector toward an expanded configuration, and (ii) a flex circuit assembly secured to the resilient frame assembly, the flex circuit assembly comprising: (A) a resilient substrate, and (B) a plurality of electrodes positioned on the resilient substrate, the plurality of electrodes being configured perform one or both of: (1) picking up electrical potentials from tissue or blood, or (2) ablating tissue.

### Example 31

The apparatus of Example 30, the resilient substrate being configured to bias the flex circuit assembly toward a flat configuration.

### Example 32

An apparatus comprising: (a) a catheter shaft assembly having a proximal end and a distal end, the catheter shaft assembly defining a longitudinal axis; and (b) an end effector associated with the distal end of the catheter shaft assembly, the end effector comprising: (i) a resilient frame assembly, the resilient frame assembly being configured to resiliently bias the end effector toward a flat, expanded configuration, and (ii) a flex circuit assembly secured to the resilient frame assembly, the flex circuit assembly comprising: (A) an extensible substrate, and (B) a plurality of electrodes positioned on the extensible substrate, the plurality of electrodes being configured perform one or both of: (1) picking up electrical potentials from tissue or blood, or (2) ablating tissue.

### Example 33

The apparatus of Example 32, the extensible substrate comprising silicone.

### Example 34

An apparatus comprising: (a) a catheter shaft assembly having a proximal end and a distal end, the catheter shaft assembly defining a longitudinal axis; and (b) an end effector associated with the distal end of the catheter shaft assembly, the end effector comprising: (i) a resilient frame assembly, the resilient frame assembly being configured to resiliently bias the end effector toward a flat, expanded configuration, and (ii) a flex circuit assembly secured to the resilient frame assembly, the flex circuit assembly comprising: (A) a flexible, non-extensible substrate, and (B) a plurality of electrodes positioned on the flexible, non-extensible substrate, the plurality of electrodes being configured perform one or both of: (1) picking up electrical potentials from tissue or blood, or (2) ablating tissue.

### Example 35

The apparatus of Example 34, the flexible, non-extensible substrate comprising one or more elongation features.

### Example 36

The apparatus of Example 35, the one or more elongation features comprising one or more serpentine curves.

### Example 37

An apparatus comprising: (a) a catheter shaft assembly having a proximal end and a distal end, the catheter shaft assembly defining a longitudinal axis; and (b) an end effector associated with the distal end of the catheter shaft assembly, the end effector comprising: (i) a resilient frame assembly, the resilient frame assembly being configured to resiliently bias the end effector toward a flat, expanded configuration, and (ii) a flex circuit assembly secured to the resilient frame assembly, the flex circuit assembly comprising: (A) a flexible substrate defining a grid shape, and (B) a plurality of electrodes positioned on the flexible substrate, the plurality of electrodes being configured perform one or both of: (1) picking up electrical potentials from tissue or blood, or (2) ablating tissue.

### Example 38

The apparatus of Example 37, the grid shape being oriented askew relative to the longitudinal axis.

### Example 39

The apparatus of any of Examples 37 through 38, the electrodes being positioned at intersections defined by the grid shape.

### Example 40

An apparatus comprising: (a) a catheter shaft assembly having a proximal end and a distal end, the catheter shaft assembly defining a longitudinal axis; and (b) an end effector associated with the distal end of the catheter shaft assembly, the end effector comprising: (i) a resilient frame assembly, the resilient frame assembly being configured to resiliently bias the end effector toward a flat, expanded configuration, and (ii) a flex circuit assembly secured to the resilient frame assembly, the flex circuit assembly comprising: (A) a flexible substrate defining a plurality of longitudinally extending portions and a plurality of zig-zag shaped portions, and (B) a plurality of electrodes positioned on the flexible substrate, the plurality of electrodes being configured perform one or both of: (1) picking up electrical potentials from tissue or blood, or (2) ablating tissue.

### Example 41

An apparatus comprising: (a) a catheter shaft assembly having a proximal end and a distal end, the catheter shaft assembly defining a longitudinal axis; and (b) an end effector associated with the distal end of the catheter shaft assembly, the end effector comprising: (i) resilient frame assembly extending from the catheter shaft and defining a closed perimeter with the catheter shaft, the resilient frame assembly being configured to resiliently bias the end effector toward a flat, expanded configuration, and (ii) a generally planar flex circuit assembly disposed inside the closed perimeter and secured at discrete locations on the resilient frame assembly, the flex circuit assembly having electrodes disposed on both sides of the planar flex circuit assembly.

### IV. Miscellaneous

It should be understood that various modes of energized tissue ablation are possible, including but not limited to RF and IRE (including monopolar or bio-polar highvoltage DC pulses) or combinations may be used depending on need, availability/ and/or preference. Accordingly, references herein to "energy" and "generators" herein shall be understood to encompass all such modalities with the scope being determined by the claims herein.

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus comprising:
(a) a catheter shaft assembly having a proximal end and a distal end, the catheter shaft assembly defining a longitudinal axis; and
(b) an end effector associated with the distal end of the catheter shaft assembly, the end effector being configured to transition between a first configuration and a second configuration, the end effector being configured to fit within an outer sheath in the first configuration, the end effector being configured to expand outwardly away from the longitudinal axis in the second configuration when exposed distally relative to the distal end of the outer sheath, the end effector comprising:
(i) a resilient frame assembly, the resilient frame assembly being configured to resiliently bias the end effector toward the second configuration, and
(ii) a first flex circuit assembly secured to the resilient frame assembly, the first flex circuit assembly comprising:
(A) a first flexible substrate, and
(B) a first plurality of electrodes positioned on the first flexible substrate, the first plurality of electrodes being configured perform one or both of:
(1) picking up electrical potentials from tissue or blood, or
(2) ablating tissue.

2. The apparatus of claim 1, the end effector being configured to define a flat, planar shape in the second configuration.

3. The apparatus of any preceding claim, the resilient frame assembly comprising nitinol.

4. The apparatus of any preceding claim, the resilient frame assembly comprising at least one resilient wire.

5. The apparatus of claim 4, the resilient frame assembly comprising a first wire and a second wire joined together.

6. The apparatus of any preceding claim, the resilient frame assembly forming a loop shape.

7. The apparatus of any preceding claim, the first flexible substrate comprising a resilient material.

8. The apparatus of any preceding claim, the first flexible substrate comprising an extensible material.

9. The apparatus of any one of claims 1-7, the first flexible substrate comprising a non-extensible material.

10. The apparatus of any preceding claim, the first flexible substrate comprising one or more elongation features.

11. The apparatus of claim 10, the one or more elongation features comprising one or more serpentine curves.

12. The apparatus of claim 10, the one or more elongation features comprising one or more zig-zag structures.

13. The apparatus of any preceding claim, the first flexible substrate comprising nitinol.

14. The apparatus of any preceding claim, the first flexible substrate comprising silicone.

15. The apparatus of any preceding claim, the end effector further comprising a second flex circuit assembly secured to the resilient frame assembly, the second flex circuit assembly comprising:
(A) a second flexible substrate, and
(B) a second plurality of electrodes positioned on the second flexible substrate, the second plurality of electrodes being configured perform one or both of:
(1) picking up electrical potentials from tissue or blood, or
(2) ablating tissue.

16. The apparatus of claim 15, the first flex circuit assembly being positioned on a first side of the end effector, the second flex circuit assembly being positioned on a second side of the end effector, the second side being opposite to the first side.

17. The apparatus of any preceding claim, the end effector further comprising a second plurality of electrodes, the first plurality of electrodes being positioned on a first side of the end effector, the second plurality of electrodes being positioned on a second side of the end effector, the second side being opposite to the first side.

18. An apparatus comprising:
(a) a catheter shaft assembly having a proximal end and a distal end, the catheter shaft assembly defining a longitudinal axis; and
(b) an end effector associated with the distal end of the catheter shaft assembly, the end effector comprising:
(i) a resilient frame assembly, the resilient frame assembly being configured to resiliently bias the end effector toward an expanded configuration, and
(ii) a flex circuit assembly secured to the resilient frame assembly, the flex circuit assembly comprising:
(A) a resilient substrate, and
(B) a plurality of electrodes positioned on the resilient substrate, the plurality of electrodes being configured perform one or both of:
(1) picking up electrical potentials from tissue or blood, or
(2) ablating tissue.

19. The apparatus of claim 18, the resilient substrate being configured to bias the flex circuit assembly toward a flat configuration.

20. An apparatus comprising:
(a) a catheter shaft assembly having a proximal end and a distal end, the catheter shaft assembly defining a longitudinal axis; and
(b) an end effector associated with the distal end of the catheter shaft assembly, the end effector comprising:
(i) a resilient frame assembly extending from the catheter shaft and defining a closed perimeter with the catheter shaft, the resilient frame assembly being configured to resiliently bias the end effector toward a flat, expanded configuration, and
(ii) a generally planar flex circuit assembly disposed inside the closed perimeter and secured at discrete locations on the resilient frame assembly, the flex circuit assembly having electrodes disposed on both sides of the planar flex circuit assembly.
